# EUROPEAN PATENT APPLICATION

(11) **EP 4 800 104 A1**
(43) Date of publication of application: **02.09.2026**
(21) Application number: 24881770.2
(22) Date of filing: 25.10.2024
(51) Int. Cl.: C12N 9/22, C12N 9/78, C12N 15/113, C12N 15/85, C12Q 1/6816, A61K 38/46, A61P 7/00, A61P 9/00

(54) **DEAMINASE, BASE EDITOR COMPRISING SAME, AND USE THEREOF**

(30) Priority: 25.10.2023 CN 202311402961
(71) Applicant: Yoltech Therapeutics Co., Ltd, Shanghai 201109 (CN)
(72) Inventor: ZHANG, Hongling, Shanghai 201109 (CN)
(74) Representative: Cabinet Becker et Associés
(86) International application number: PCT/CN2024/127500
(87) International publication number: WO 2025/087411

(57) **Abstract**

Disclosed are a deaminase, a base editor comprising same, and the use thereof. The deaminase comprises the following sequence: (i) an amino acid sequence as shown in SEQ ID NO: 10; or (ii) an amino acid sequence having at least 80% sequence identity to the amino acid sequence as shown in SEQ ID NO: 10. The provided deaminase can improve editing efficiency when formed into the base editor and used in a base editing system, and has prospects for clinical application.

## Description

This application claims priority to Chinese Patent Application No. 2023114029610 filed on October 25, 2023. The entire content of the aforesaid Chinese patent application is incorporated herein by reference.

### TECHNICAL FIELD

The present disclosure belongs to the field of gene editing, and specifically relates to a deaminase, a base editor comprising the same, and uses thereof.

### BACKGROUND

How to perform precise and efficient modification on the genome is an important objective in the study of the field of life sciences. Conventional CRISPR/Cas9 technology introduces DNA Double Strand Breaks (DSBs) at target loci, thereby triggering intracellular repair pathways including Homologous Recombination (HR) and Non-Homologous End Joining (NHEJ), so as to achieve the modification on genomic DNA, such as site-directed knockout, substitution, insertion, etc. Nevertheless, DNA repair initiated by DSBs can hardly achieve efficient and stable single-base mutations.

Currently available base editors include cytidine base editors (e.g., BE4) that convert a target C·G base pair to T·A, and adenine base editors (e.g., ABE8e) that convert A·T to G·C. For applications requiring higher editing efficiency, the use of existing base editors may be limited by their editing efficiency. There is a demand in the art for base editors with higher specificity and editing efficiency, and it is highly necessary to improve the base editors.

### SUMMARY

The technical problem to be solved by the present disclosure is that the prior art lacks base editors with higher specificity and editing efficiency, and the present disclosure provides a deaminase, a base editor comprising the same, and uses thereof. The deaminase provided by the present disclosure can improve editing efficiency when used in constructing a base editor and in a base editing system, and has prospects for clinical application.

The present disclosure solves the above technical problem by the following technical solutions.

In a first aspect, the present disclosure provides a deaminase comprising the following sequence:
(i) the amino acid sequence as set forth in SEQ ID NO: 10; or
(ii) an amino acid sequence having at least 80%, 82%, 85%, 87%, 90%, 92%, 95%, 96%, 97%, 98% or 99% sequence identity to the amino acid sequence as set forth in SEQ ID NO: 10, which retains the deaminase activity of the deaminase having the amino acid sequence as set forth in SEQ ID NO: 10; and which is not SEQ ID NO: 1.

In some embodiments, the amino acid sequence having at least 80%, 82%, 85%, 87%, 90%, 92%, 95%, 96%, 97%, 98% or 99% sequence identity to the amino acid sequence as set forth in SEQ ID NO: 10 is an amino acid sequence obtained by addition, substitution, deletion or insertion of one or more amino acid residues in the amino acid sequence as set forth in SEQ ID NO: 10.

In some embodiments, the substitution occurs at one or more of the following positions in the amino acid sequence as set forth in SEQ ID NO: 10:
C46, Y47, G48, H49, C144, Q145, F146, Y147, Q148, Q149, P150, R151, E152, V153, F154, N155, A156, E157, R158, E159, A160, R161, R162, L163, N164, Q165, P166, D167, R168, A169 and D170.

In some preferred embodiments, the substitution is a substitution occurring in a combination of the following positions in the amino acid sequence as set forth in SEQ ID NO: 10:
(1) 7 or 10 of C46, Y47, G48, H49, Q148, P150, E152, V153, F154 and N155;
(2) 6, 7 or 8 of Q148, Q149, P150, R151, E152, V153, F154 and N155;
(3) 7 or 8 of A156, E157, R158, E159, A160, R161, R162 and L163;
(4) 6 or 7 of N164, Q165, P166, D167, R168, A169 and D170;
(5) 6 or 7 of C144, Q145, F146, Y147, Q148, Q149 and P150; or
(6) 2 or 9 of C144, Q145, Q148, Q149, P150, E152, V153, F154 and N155.

In some preferred embodiments, the substitution is a substitution occurring in any one of the combination of the following positions in the amino acid sequence as set forth in SEQ ID NO: 10:
(1) C46, Y47, G48, H49, Q148, P150, E152, V153, F154 and N155;
(2) G48, Q148, P150, E152, V153, F154 and N155;
(3) Q148, Q149, P150, E152, V153, F154 and N155;
(4) Q148, P150, E152, V153, F154 and N155;
(5) Q149, P150, R151, E152, V153, F154 and N155;
(6) Q148, Q149, P150, R151, E152, V153, F154 and N155;
(7) Q148, Q149, P150, E152, F154 and N155;
(8) A156, E157, R158, E159, A160, R161, R162 and L163;
(9) A156, E157, R158, E159, A160, R162 and L163;
(10) N164, Q165, P166, D167, R168, A169 and D170;
(11) N164, Q165, D167, R168, A169 and D170;
(12) C144, Q145, F146, Y147, Q148, Q149 and P150;
(13) C144, Q145, F146, Y147, Q148 and P150;
(14) C144, Q145, Q148, Q149, P150, E152, V153, F154 and N155;
(15) C144 and Q145.

In some specific embodiments, the substitution occurring at said positions is selected from: C46P, Y47I, G48A/T, H49R, C144T/L/W, Q145L/K, F146A/R, Y147S/F, Q148R/G/T/S/C, Q149N/P/R/V/G/F/C/K, P150A/L/R/I/G/S/T, R151K/P, E152P/L/Q/H/S, V153T/A/F/Y/K/P, F154S/P/V/N/L/D/H, N155P/G/T/S/Y/R/A, A156L/T, E157F, R158N/L, E159L/H, A160K/T, R161K, R162L/K, L163D/I, N164G/R, Q165T/L, P166Q, D167L, R168L/P, A169N/T and D170R/H.

In the present disclosure, the symbol "/" denotes that the alternatives before and after the symbol are alternative embodiments. For example, C144T/L/W means that the C at position 144 may be substituted with T, L or W.

In some specific embodiments, the substitution occurring at said positions is selected from: C46P, Y47I, G48A/T, H49R, C144T/L/W, Q145L/K, F146A/R, Y147S/F, Q148R/T/C, Q149N/P/R/G/C/K, P150A/L/R/G/S/T, R151P, E152P/L/Q/H, V153T/A/F/K, F154S/P/V/L/D/H, N155P/G/T/S/A, A156L/T, E157F, R158N/L, E159L/H, A160K/T, R161K, R162L/K, L163D/I, N164G/R, Q165T/L, P166Q, D167L, R168L/P, A169N/T and D170R/H.

In some specific embodiments, the substitution is a substitution occurring in a combination of the following positions in the amino acid sequence as set forth in SEQ ID NO: 10:
(1) Q148R, Q149N, P150A, E152P, V153T, F154S, N155P;
(2) Q148R, P150L, E152L, V153A, F154P, N155G;
(3) Q148R, Q149R, P150R, E152P, V153F, F154V, N155T;
(4) Q148G, Q149G, P150I, E152L, V153Y, F154N, N155S;
(5) Q149P, P150G, R151K, E152Q, V153K, F154L, N155P;
(6) Q148R, Q149V, P150S, R151P, E152L, V153F, F154P, N155Y;
(7) Q148T, Q149G, P150R, E152H, V153A, F154D, N155S;
(8) Q148S, Q149F, P150L, E152S, V153P, F154L, N155R;
(9) Q148G, Q149C, P150S, E152P, F154H, N155A;
(10) A156L, E157F, R158N, E159L, A160K, R161K, R162L, L163D;
(11) A156T, E157F, R158L, E159H, A160T, R162K, L163I;
(12) N164G, Q165T, P166Q, D167L, R168L, A169N, D170R;
(13) N164R, Q165L, D167L, R168P, A169T, D170H;
(14) C144T, Q145L, F146A, Y147S, Q148R, Q149K, P150S;
(15) C144L, Q145L, F146R, Y147F, Q148C, P150T;
(16) C144W, Q145K, Q148R, Q149R, P150R, E152P, V153F, F154V, N155T;
(17) C144W, Q145K;
(18) G48A, Q148R, P150L, E152L, V153A, F154P, N155G;
(19) C46P, Y47I, G48T, H49R, Q148R, P150L, E152L, V153A, F154P, N155G.

In a second aspect, the present disclosure provides a base editor fusion protein, which comprises the deaminase according to the first aspect, and a nucleic acid-programmable nucleotide-binding domain.

In some embodiments, said nucleic acid-programmable nucleotide-binding domain is a Cas protein or an AGO protein.

In some embodiments, said Cas protein is selected from Cas9, CasX, CasY, Cpf1, C2c1, C2c2 and C2c3.

In some embodiments, said AGO protein is selected from pAgo, eAgo, Ago1, Ago2, Ago3 and Ago4.

In some embodiments, said deaminase is linked to one terminus of said nucleic acid-programmable nucleotide-binding domain or is chimeric within said nucleic acid-programmable nucleotide-binding domain.

In some preferred embodiments, said linkage is a direct linkage or a linkage via a linker. Said linker preferably comprises an amino acid sequence as set forth in one or more of SEQ ID NOs: 32-41.

In some preferred embodiments, said chimeric site is located in the carboxy-terminal domain of said nucleic acid-programmable nucleotide-binding domain.

In some preferred embodiments, said nucleic acid-programmable nucleotide-binding domain retains part or none of the cleavage activity on a nucleotide strand.

In some embodiments, said base editor fusion protein further comprises a nuclear localization signal (NLS) sequence; said NLS sequence is linked to the N-terminus and/or C-terminus of said base editor fusion protein, and/or to the N-terminus and/or C-terminus of said deaminase.

In some preferred embodiments, said NLS sequence is linked to both the N-terminus and the C-terminus of said base editor fusion protein.

In some specific embodiments, the structure of said base editor fusion protein from N-terminus to C-terminus is: NLS sequence-deaminase-nucleic acid-programmable nucleotide-binding domain-NLS sequence.

In other specific embodiments, the structure of said base editor fusion protein from N-terminus to C-terminus is: NLS sequence-deaminase-nucleic acid-programmable nucleotide-binding domain-NLS sequence.

In some specific embodiments, when said nucleic acid-programmable nucleotide-binding domain is a Cas protein, e.g., a Cas9 protein, said chimeric site is located between positions 1249 and 1250 of Cas9.

In some specific embodiments, said base editor fusion protein comprises an amino acid sequence as set forth in any one of SEQ ID NOs: 10, 18, 20 and 22.

In a third aspect, the present disclosure provides a base editing system, which comprises:
(i) the deaminase according to the first aspect and a nucleic acid-programmable nucleotide-binding domain;
or (ii) the base editor fusion protein according to the second aspect,
and a guide polynucleotide;
wherein said nucleic acid-programmable nucleotide-binding domain or said base editor fusion protein forms a ribonucleoprotein complex with said guide polynucleotide, and binds to a target nucleic acid under the guidance of said guide polynucleotide.

In a fourth aspect, the present disclosure provides a polynucleotide encoding the deaminase according to the first aspect, the base editor fusion protein according to the second aspect, or the base editing system according to the third aspect.

In some specific embodiments, the polynucleotide encoding said base editor fusion protein comprises a nucleotide sequence as set forth in any one of SEQ ID NOs: 11, 19, 21 and 23.

In a fifth aspect, the present disclosure provides a vector comprising the polynucleotide according to the fourth aspect.

In some embodiments of the present disclosure, said polynucleotide is located on one or more vectors.

In some embodiments, said polynucleotide is operably linked to a promoter.

In some embodiments, said promoter is one or more selected from a constitutive promoter, an inducible promoter, a ubiquitin promoter, a cell type-specific promoter and a tissue-specific promoter.

In a sixth aspect, the present disclosure provides an isolated cell comprising the deaminase according to the first aspect, the base editor fusion protein according to the second aspect, the polynucleotide according to the fourth aspect, and/or the vector according to the fifth aspect.

In some embodiments, said cell is a prokaryotic cell or a eukaryotic cell; e.g., selected from an animal cell, a plant cell and a fungal cell.

In some preferred embodiments, said cell is a vertebrate cell or an invertebrate cell; said vertebrate cell is preferably a mammalian cell.

In some preferred embodiments, said mammalian cell is selected from a rodent cell, a primate cell and a non-primate cell; said primate cell is e.g., a human cell.

In a seventh aspect, the present disclosure provides a pharmaceutical composition comprising the deaminase according to the first aspect, the base editor fusion protein according to the second aspect, the base editing system according to the third aspect, the polynucleotide according to the fourth aspect, the vector according to the fifth aspect, and/or the cell according to the sixth aspect, and optionally a pharmaceutically acceptable carrier and/or excipient.

In an eighth aspect, the present disclosure provides a kit comprising the deaminase according to the first aspect, the base editor fusion protein according to the second aspect, the base editing system according to the third aspect, the polynucleotide according to the fourth aspect, the vector according to the fifth aspect, the cell according to the sixth aspect, and/or the pharmaceutical composition according to the seventh aspect.

In a ninth aspect, the present disclosure provides a delivery system comprising the deaminase according to the first aspect, the base editor fusion protein according to the second aspect, the base editing system according to the third aspect, the polynucleotide according to the fourth aspect, the vector according to the fifth aspect, the cell according to the sixth aspect, the pharmaceutical composition according to the seventh aspect, and/or the kit according to the eighth aspect.

In some embodiments, the delivery vehicle is selected from liposomes, nanoparticles, viral vectors, exosomes, microvesicles and cell-penetrating peptides.

In a tenth aspect, the present disclosure provides a method for base editing, comprising the step of contacting the deaminase according to the first aspect, the base editor fusion protein according to the second aspect, or the base editing system according to the third aspect with a target nucleic acid to effect a deamination reaction.

In some embodiments, the method for base editing is performed *in vivo* or *in vitro*.

In some embodiments, the method for base editing is for non-diagnostic or non-therapeutic purposes.

In an eleventh aspect, the present disclosure provides use of the deaminase according to the first aspect, the base editor fusion protein according to the second aspect, the base editing system according to the third aspect, the polynucleotide according to the fourth aspect, the vector according to the fifth aspect, the cell according to the sixth aspect, the pharmaceutical composition according to the seventh aspect, the kit according to the eighth aspect, or the delivery system according to the ninth aspect in the manufacture of a medicament for treating a disease associated with or caused by a point mutation.

In some embodiments, the disease is one or more selected from hypercholesterolemia, transthyretin amyloidosis, alpha-1 antitrypsin deficiency, and beta-hemoglobinopathies.

In a twelfth aspect, the present disclosure provides a method of treating a condition or disease, comprising administering to a subject in need thereof an effective amount of the deaminase according to the first aspect, the base editor fusion protein according to the second aspect, the base editing system according to the third aspect, the polynucleotide according to the fourth aspect, the vector according to the fifth aspect, the cell according to the sixth aspect, the pharmaceutical composition according to the seventh aspect, the kit according to the eighth aspect, and/or the delivery system according to the ninth aspect.

In a thirteenth aspect, the present disclosure provides a use of the deaminase according to the first aspect, the base editor fusion protein according to the second aspect, the base editing system according to the third aspect, the polynucleotide according to the fourth aspect, the vector according to the fifth aspect, the cell according to the sixth aspect, the pharmaceutical composition according to the seventh aspect, the kit according to the eighth aspect, or the delivery system according to the ninth aspect as a medicament.

In a fourteenth aspect, the present disclosure provides a use of the deaminase according to the first aspect, the base editor fusion protein according to the second aspect, the base editing system according to the third aspect, the polynucleotide according to the fourth aspect, the vector according to the fifth aspect, the cell according to the sixth aspect, the pharmaceutical composition according to the seventh aspect, the kit according to the eighth aspect, or the delivery system according to the ninth aspect in treating a condition or disease.

In some embodiments, the condition or disease is associated with one or more C>A point mutations or C>T point mutations; preferably, the condition or disease includes a disease as shown in the following table:

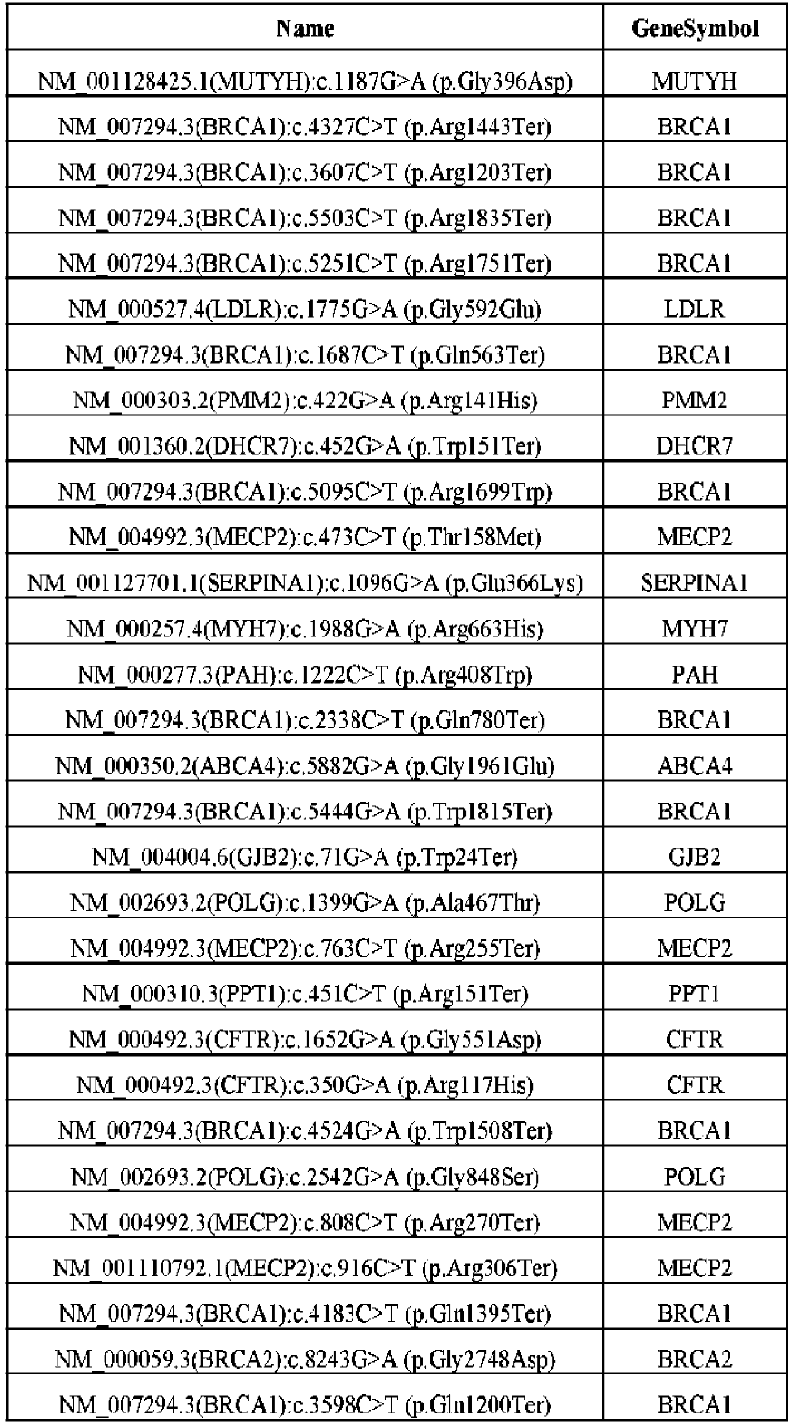

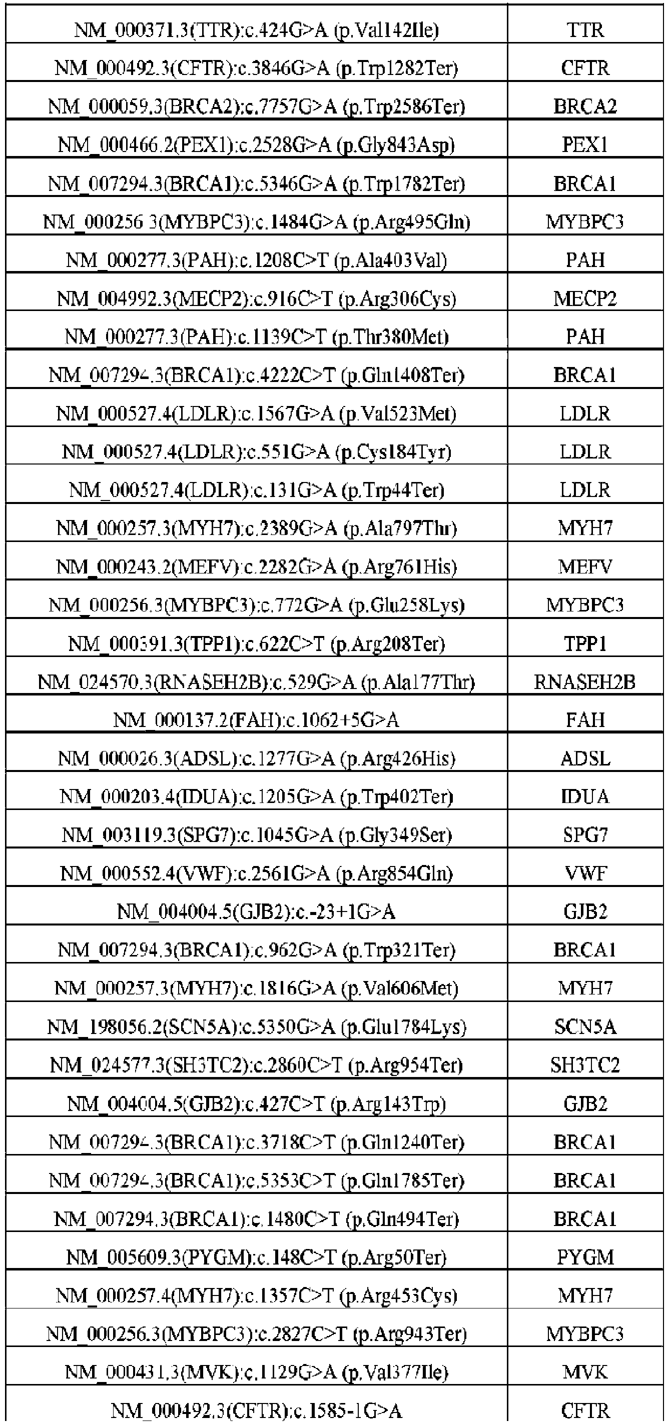

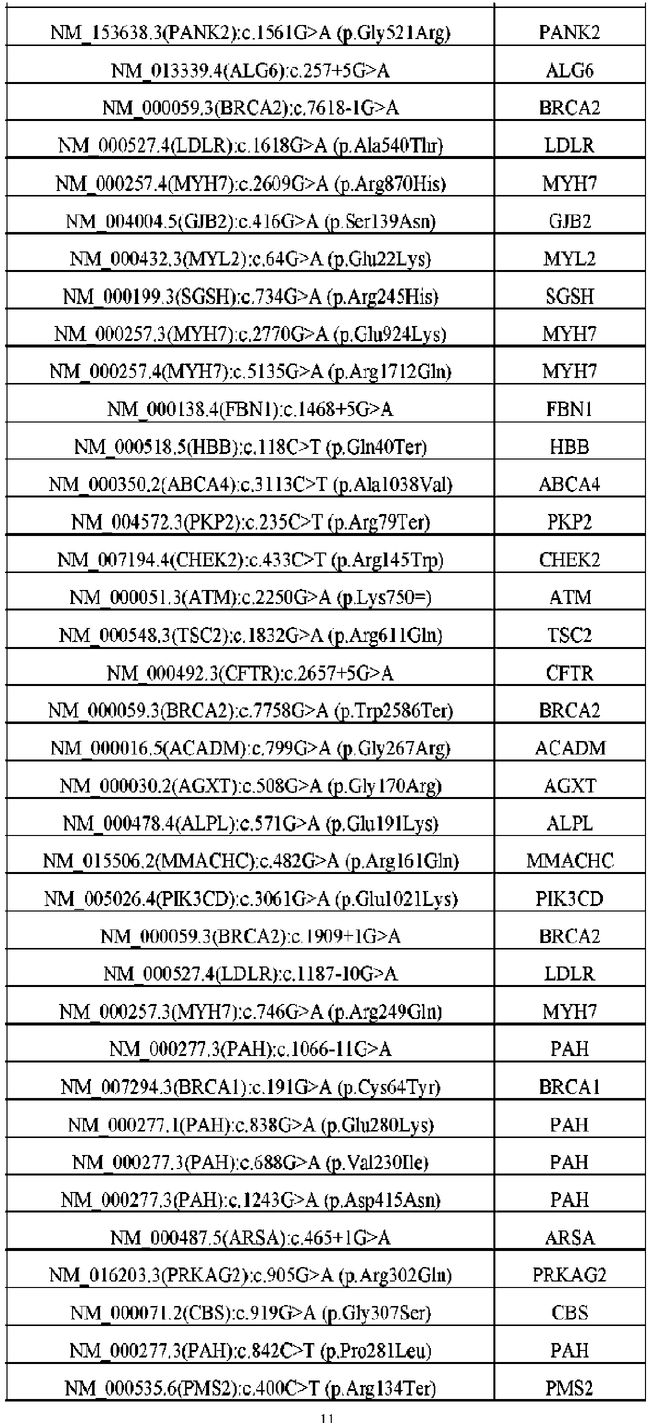

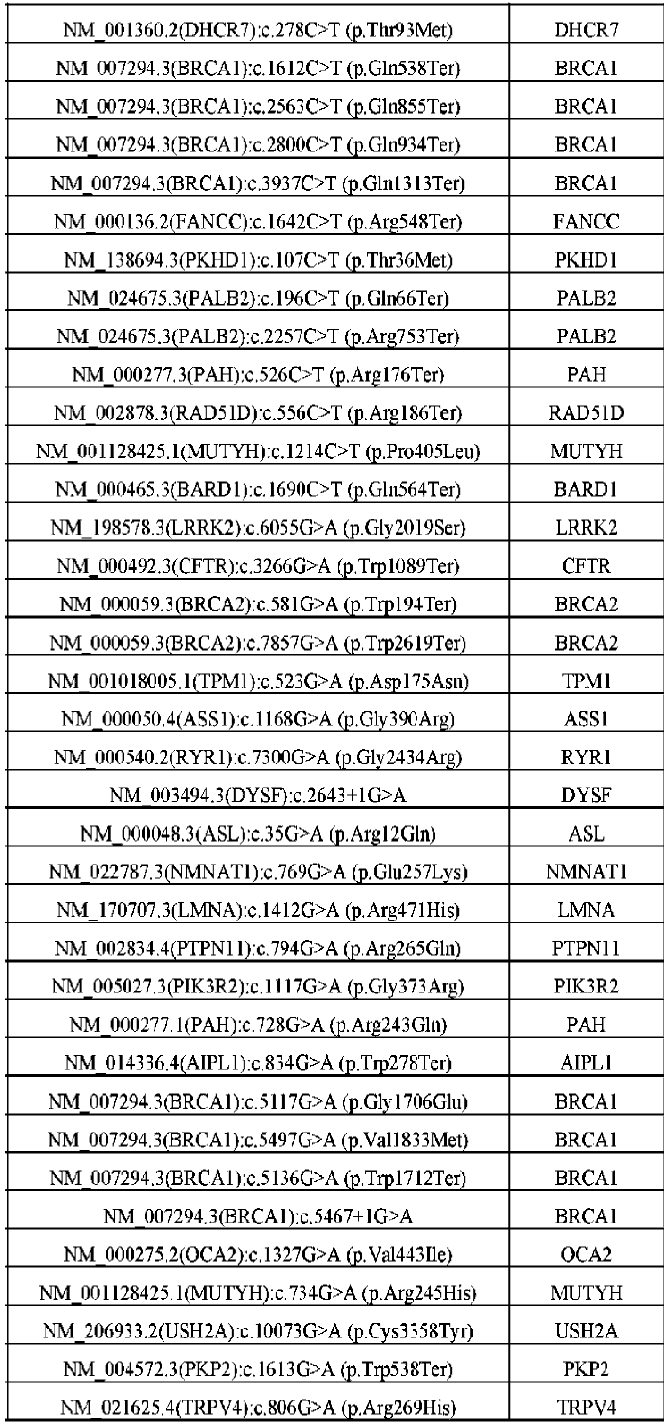

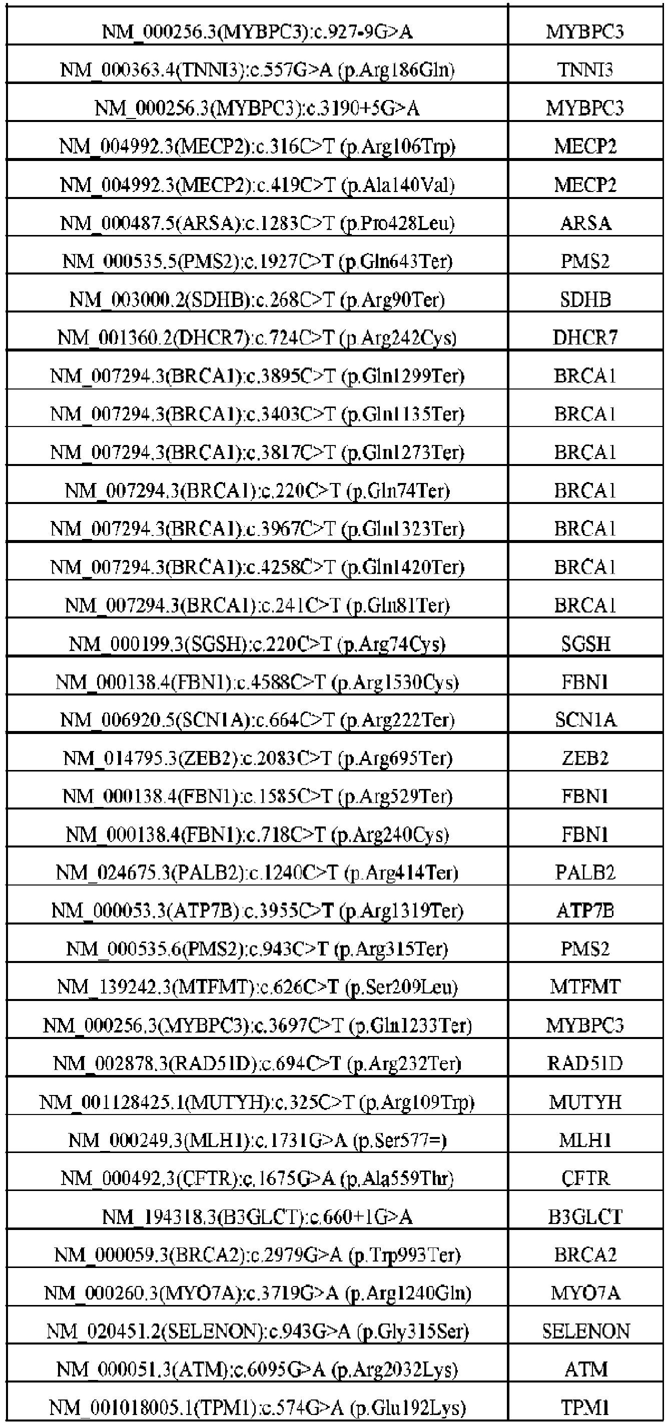

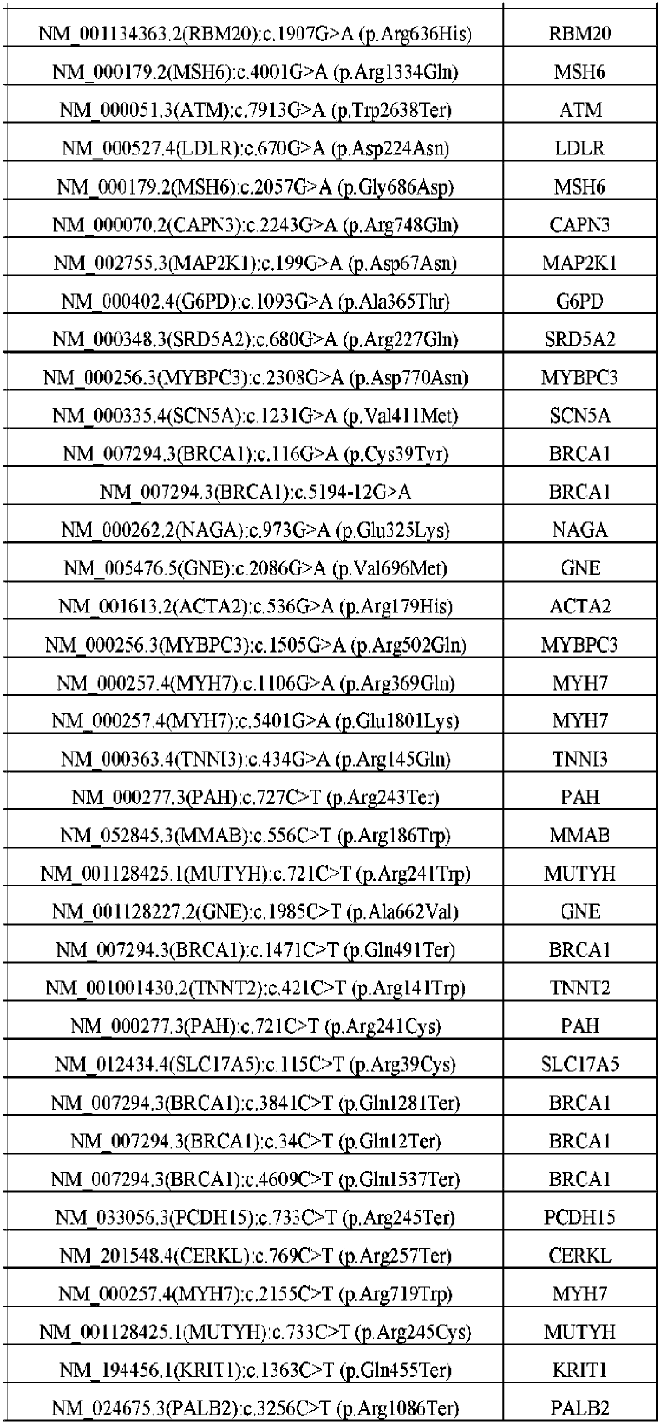

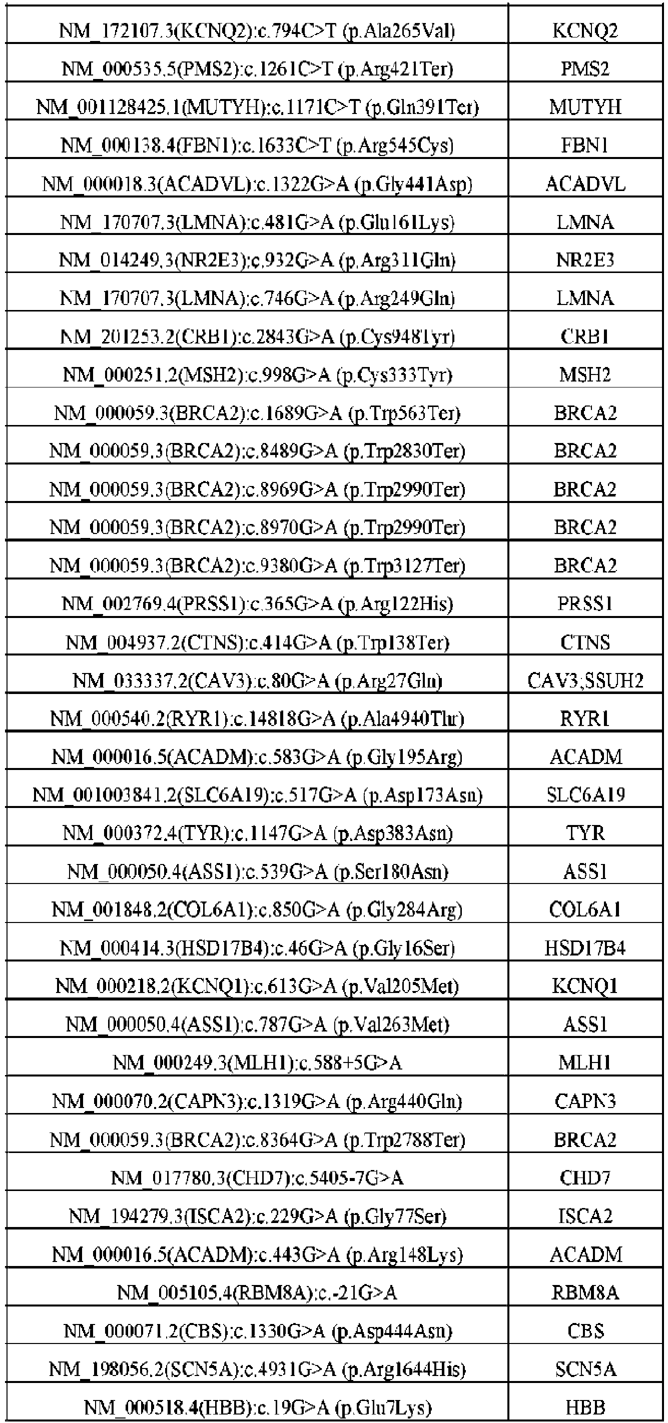

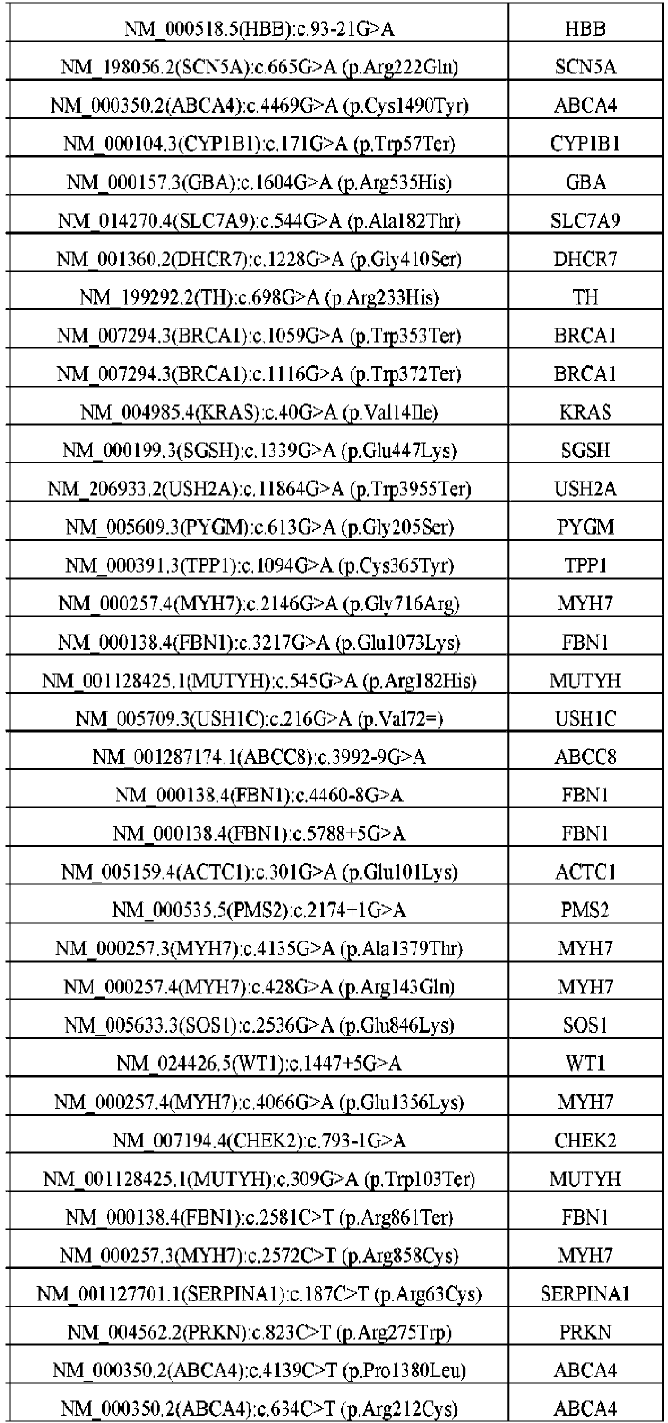

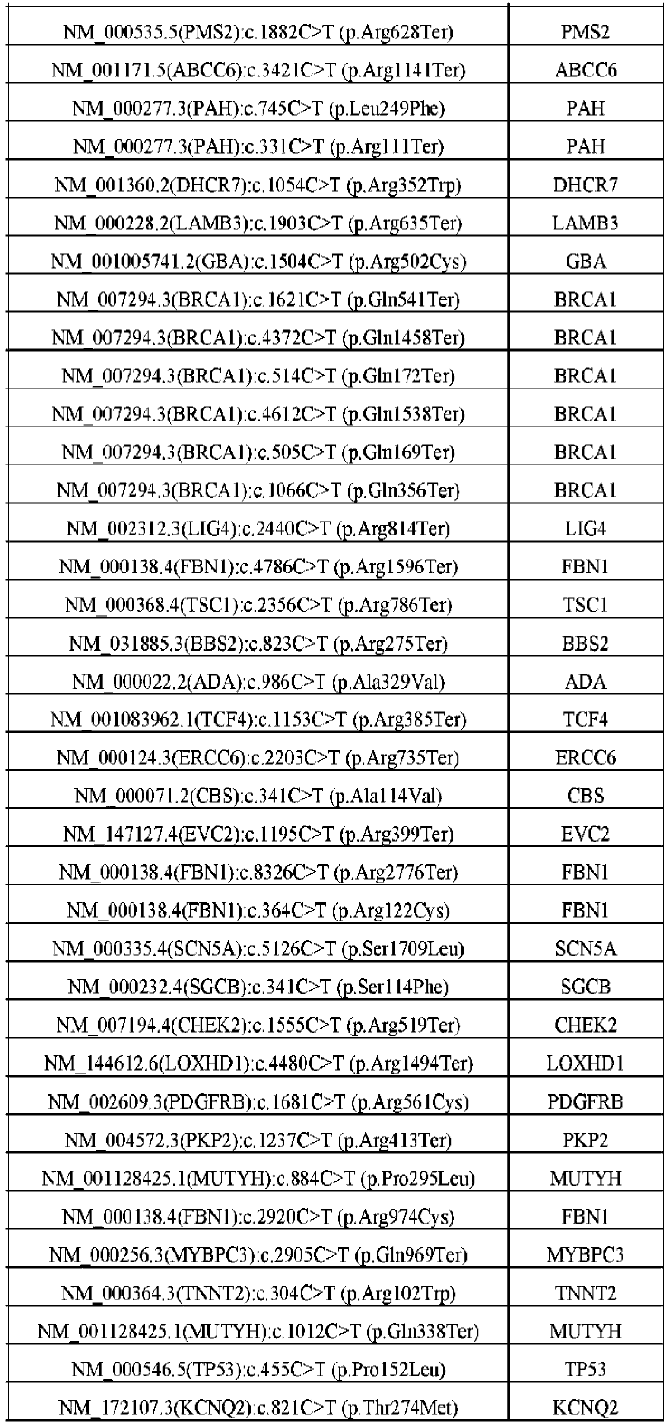

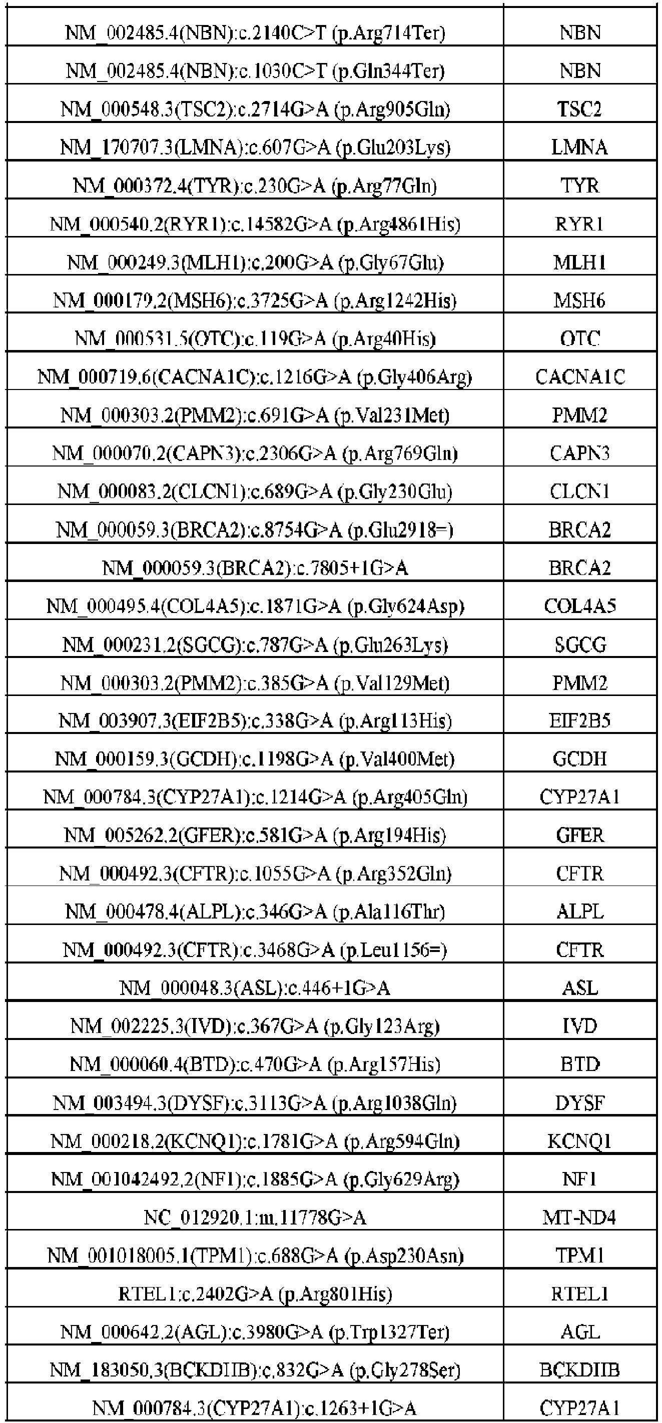

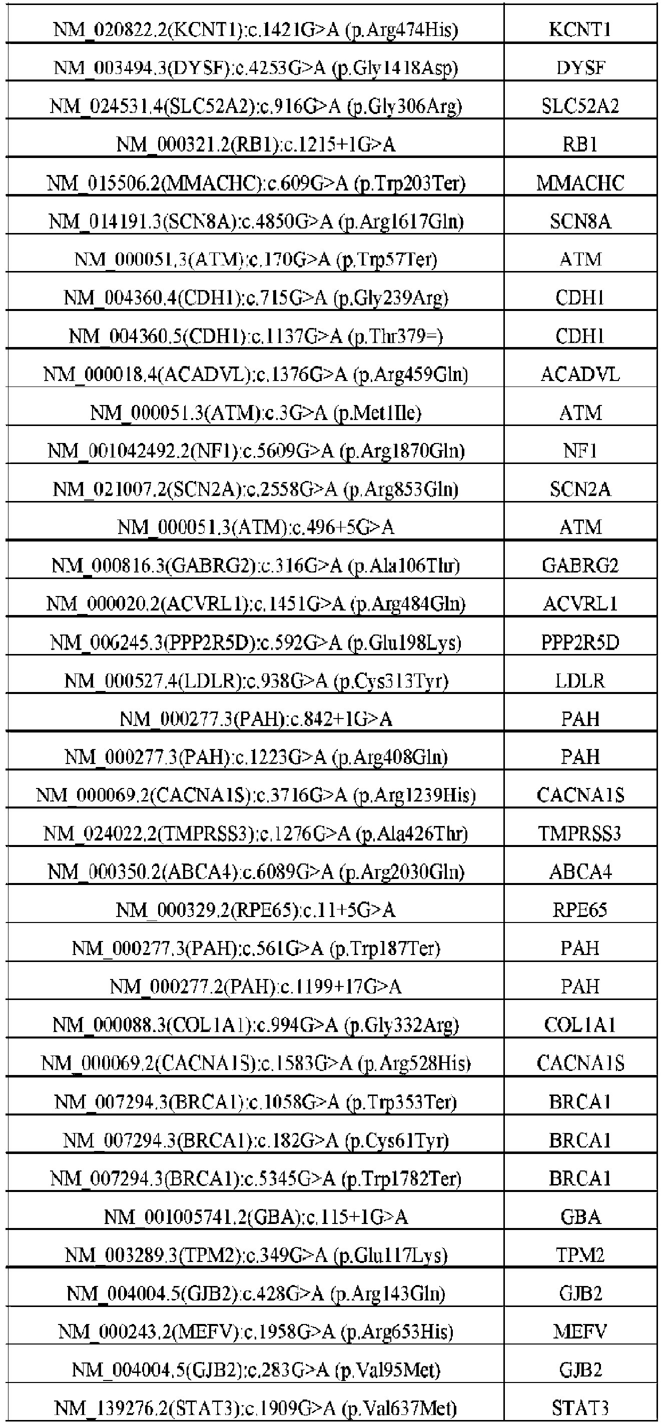

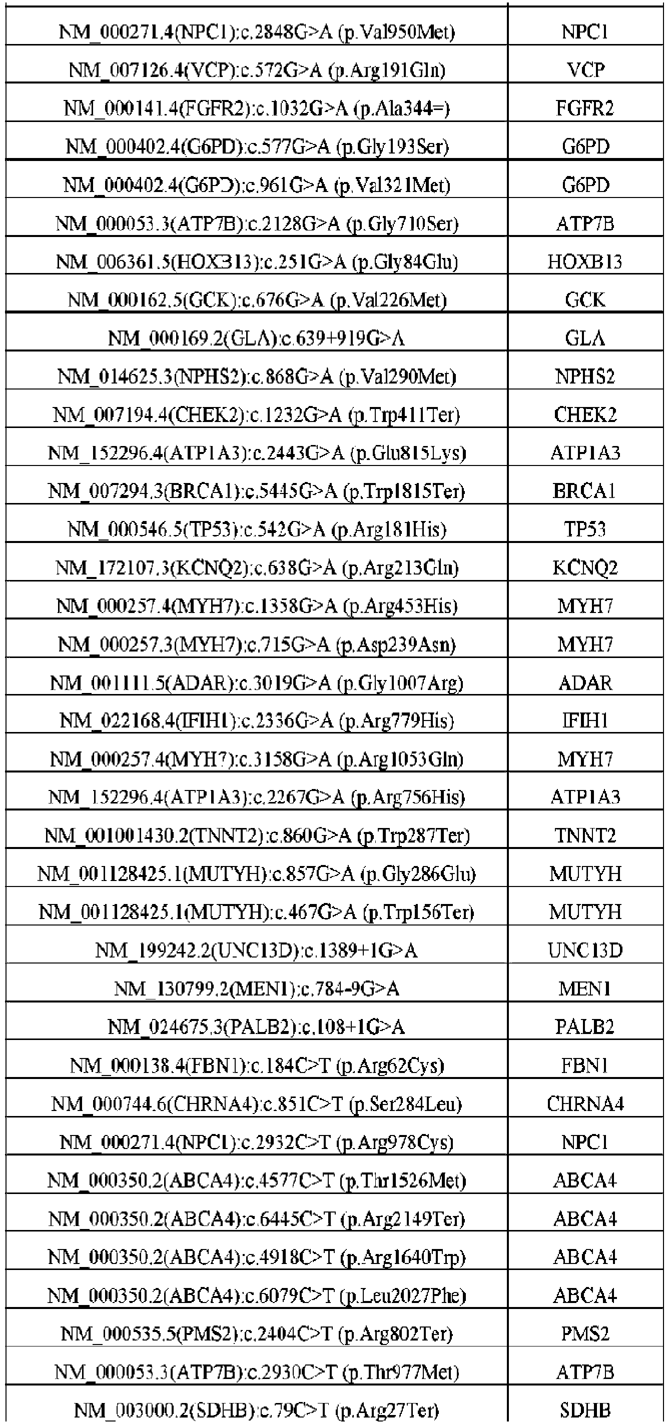

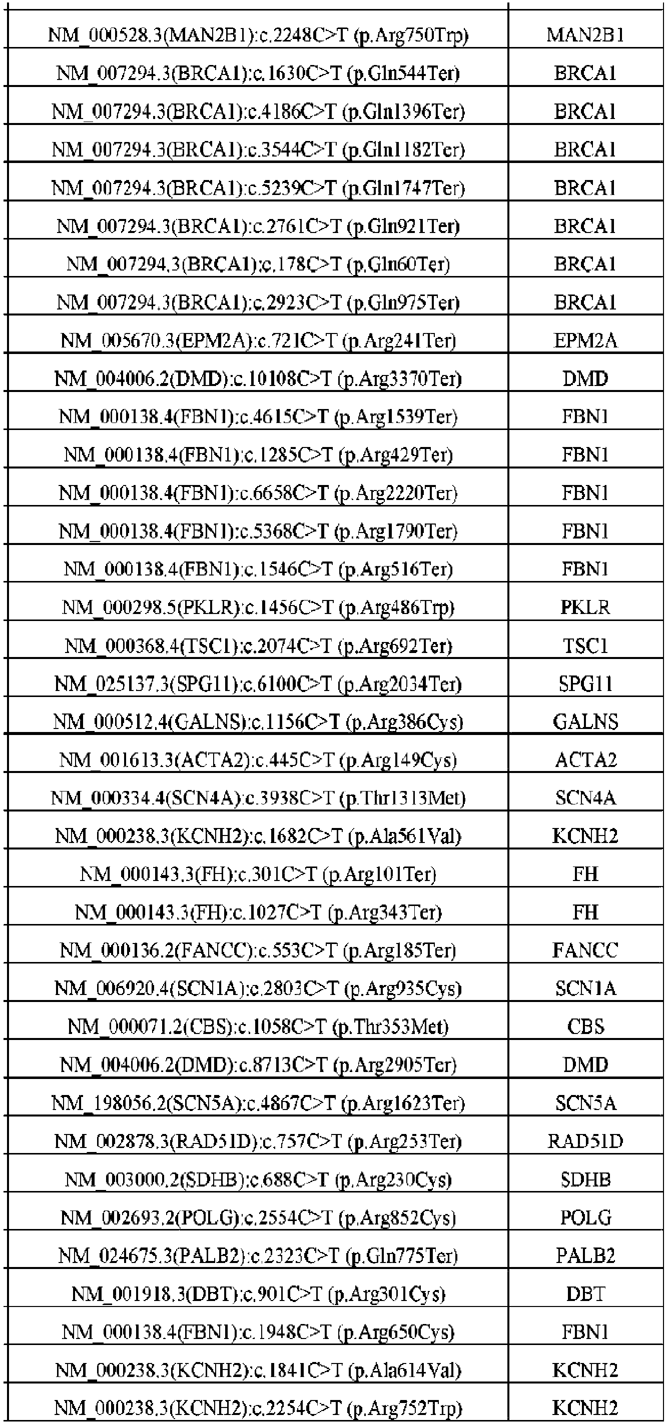

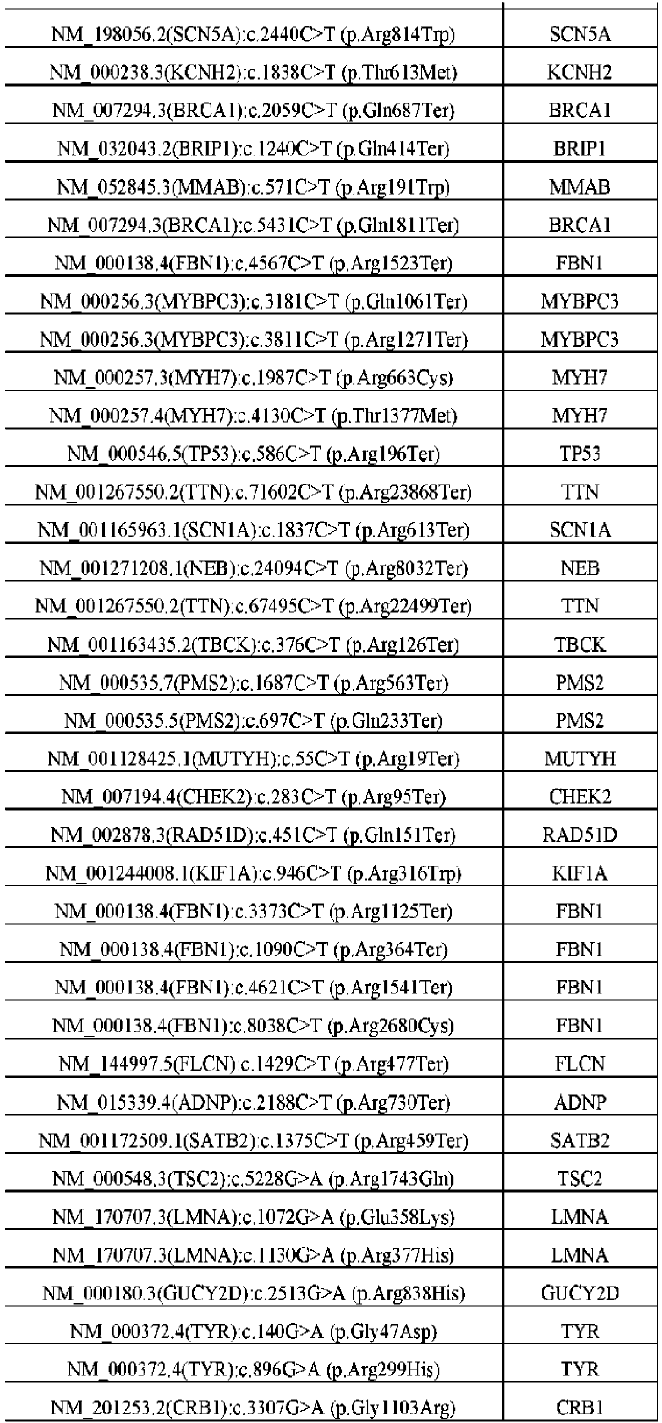

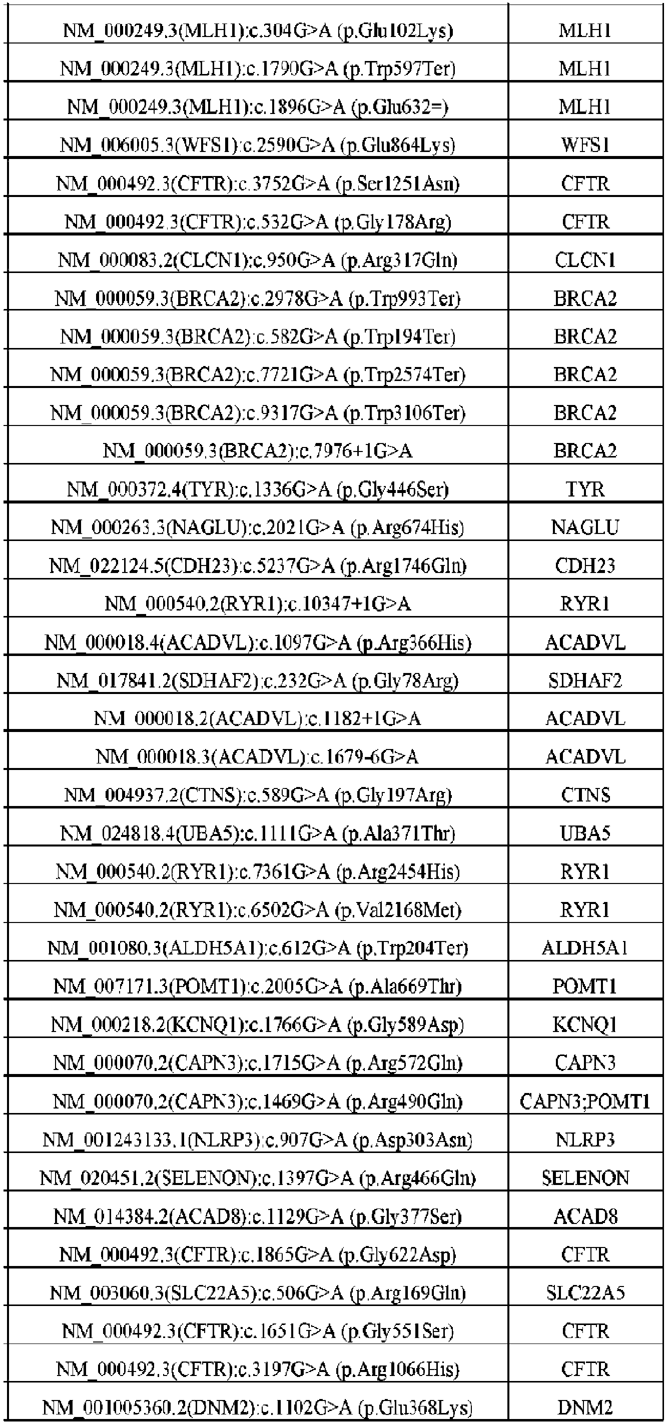

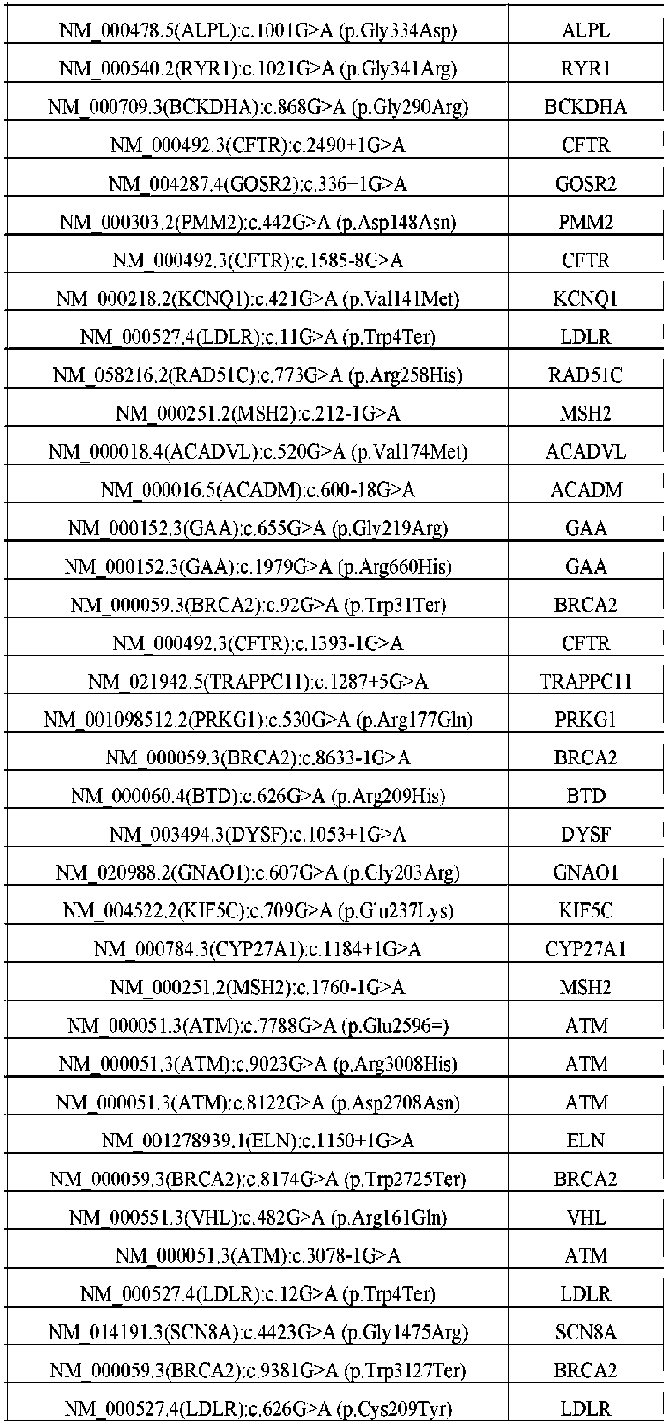

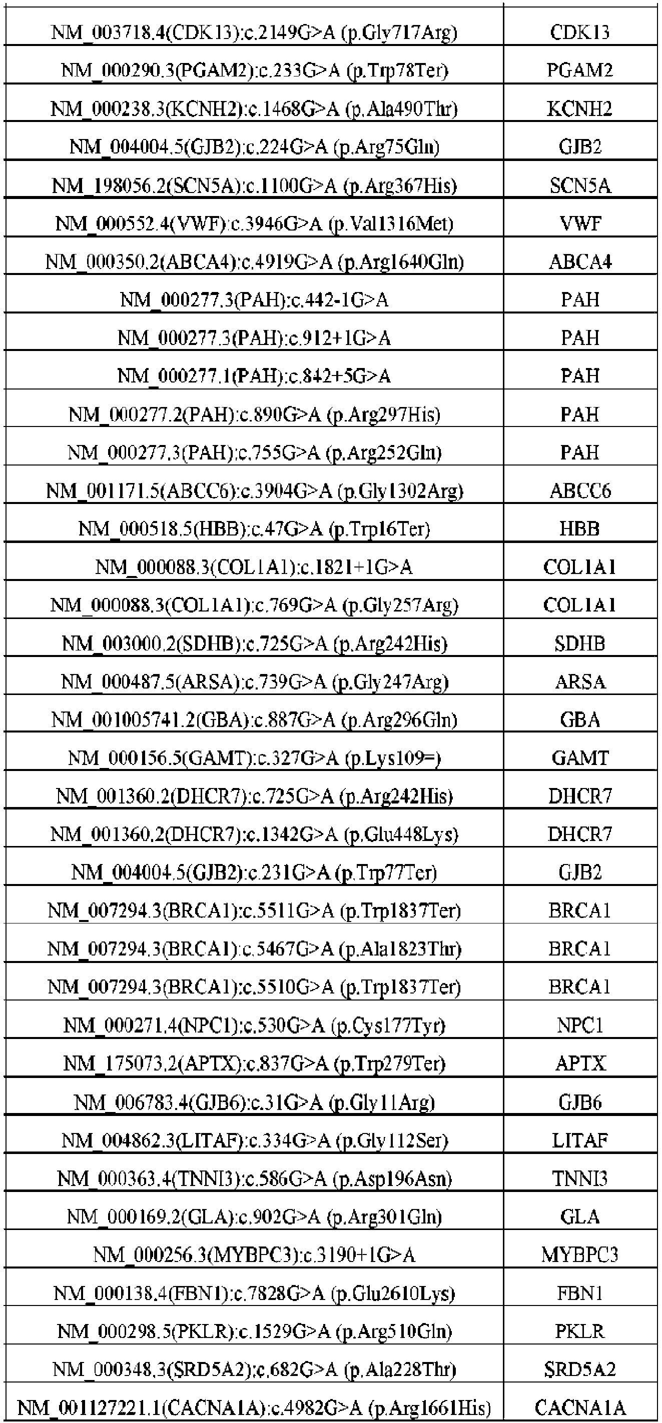

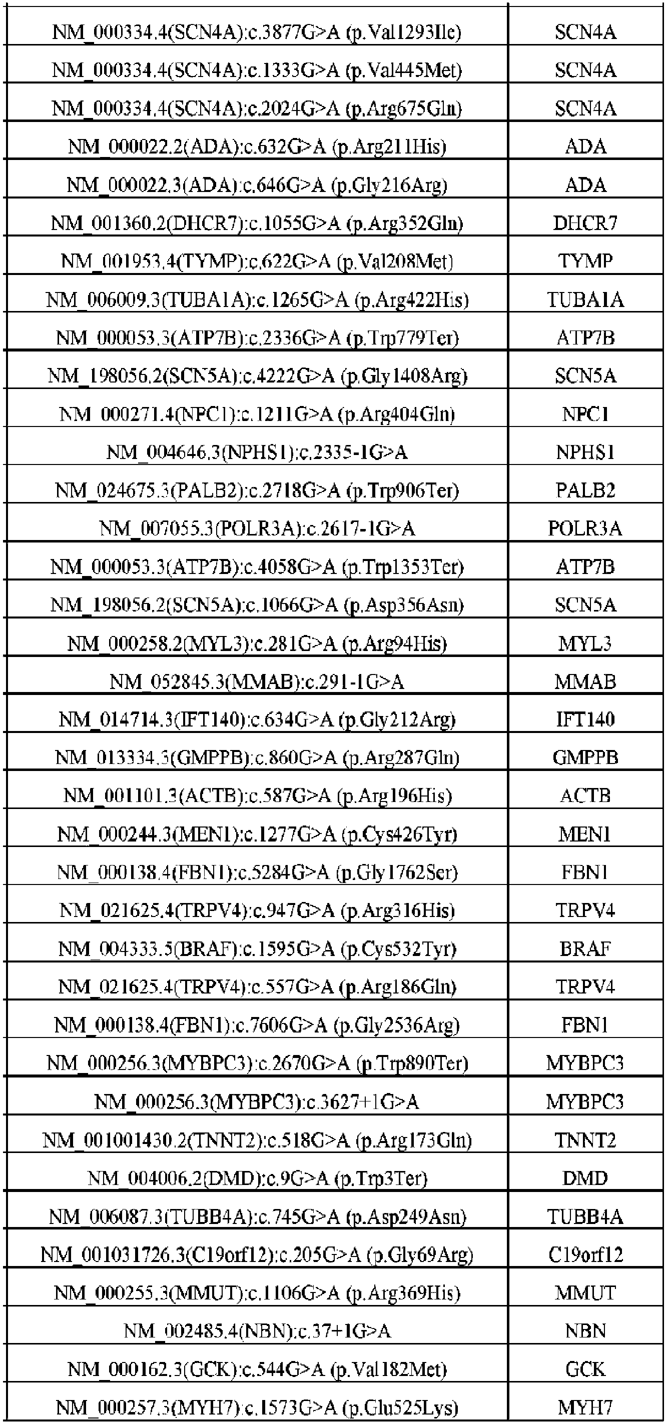

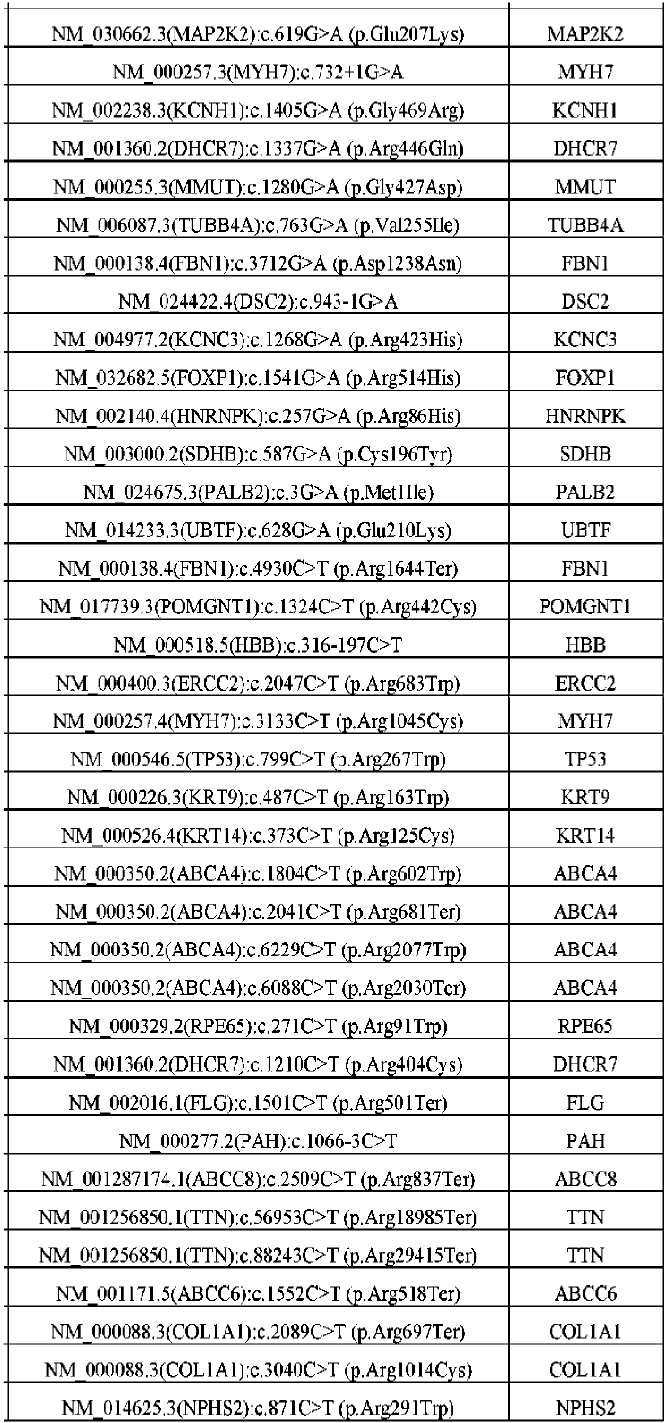

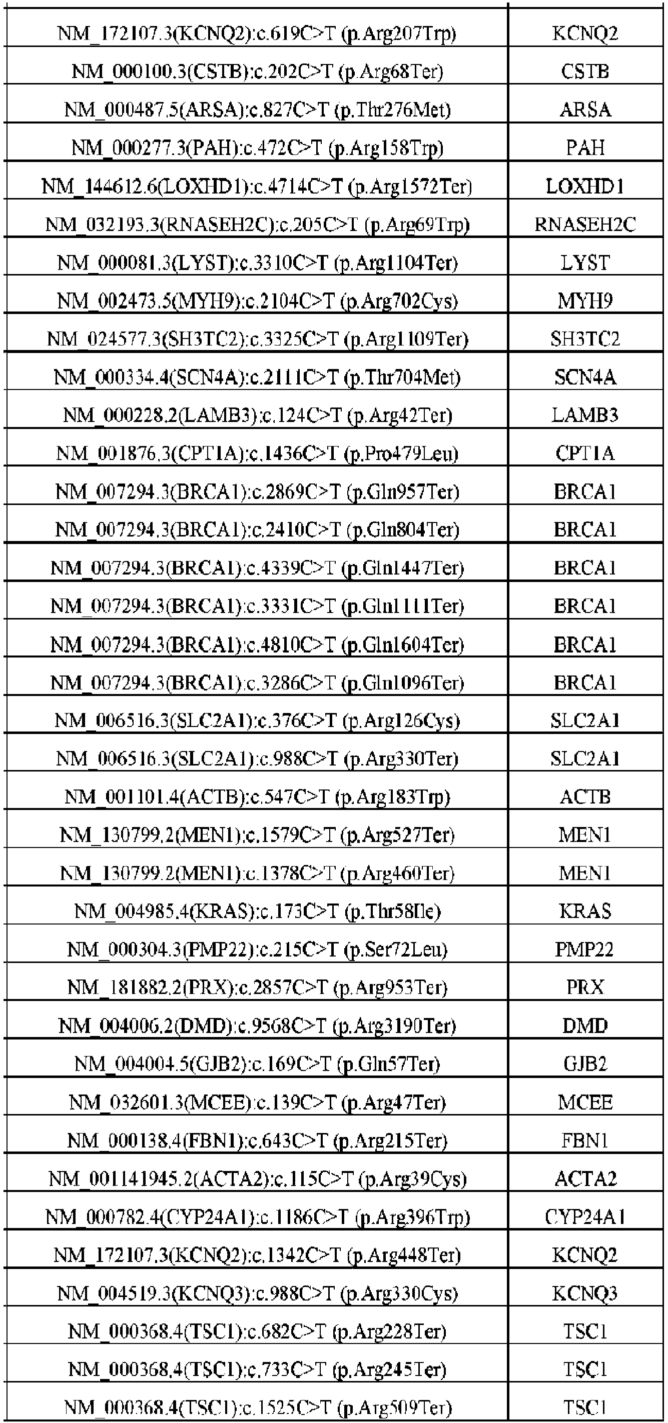

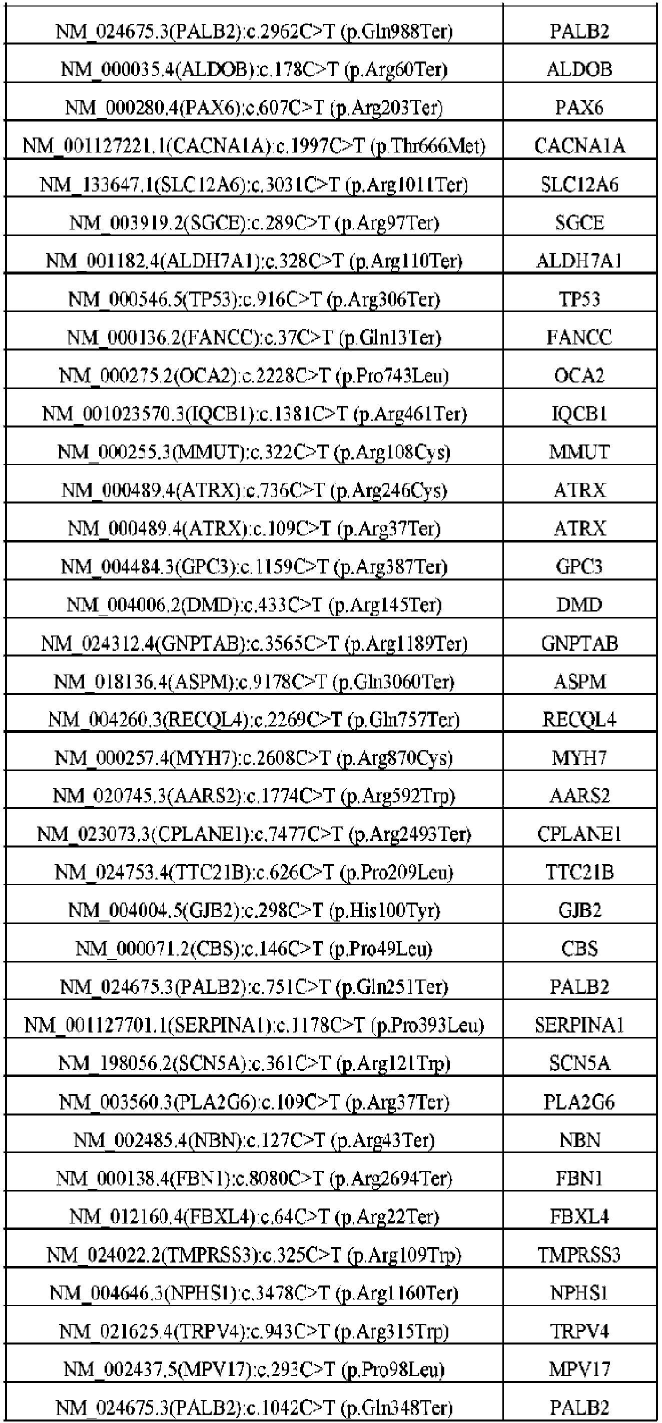

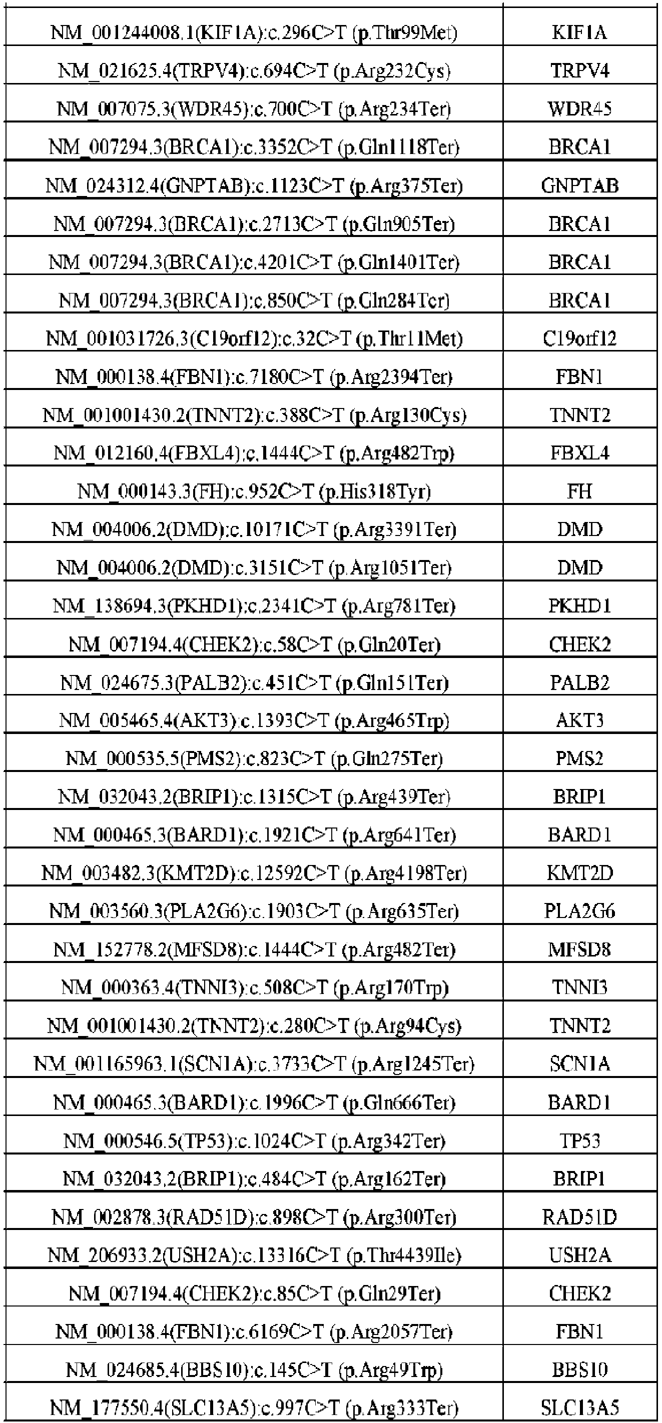

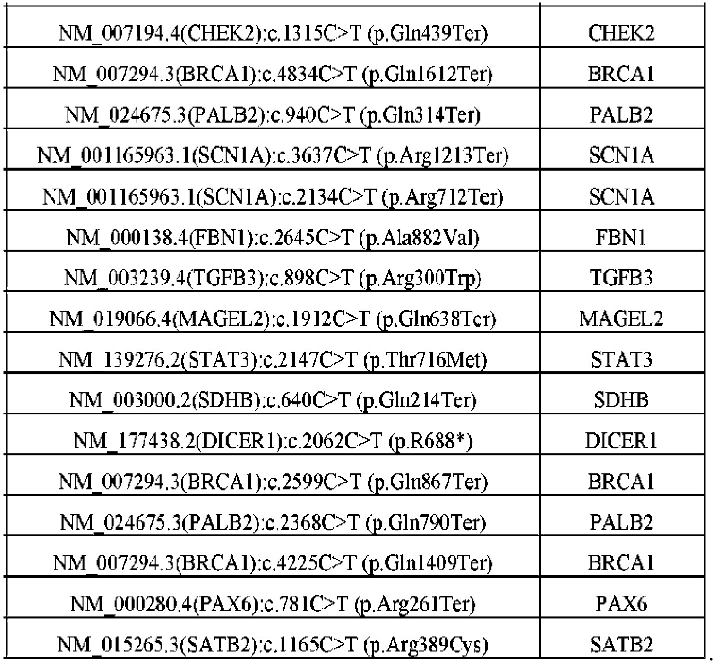

In some embodiments, the disease or condition comprises one or more of hypercholesterolemia, transthyretin amyloidosis, alpha-1 antitrypsin deficiency, and beta-hemoglobinopathies.

On the basis of conforming to common general knowledge in the art, the above preferred conditions can be combined arbitrarily to obtain preferred embodiments of the present disclosure.

The reagents and raw materials used in the present disclosure are all commercially available.

The positive and progressive effects of the present disclosure are as follows:
The deaminase provided by the present disclosure exhibits significantly improved editing efficiency when used to construct a base editor and in a base editing system, and can be used to modify pathogenic DNA target sites. For example, the base editor can be used to site-directly mutate adenine (A) to guanine (G) in a nucleic acid (e.g., DNA). Such alterations change the amino acid sequence of a protein, so as to disrupt or create a new initiation codon, or create a stop codon, to disrupt a splice donor, to disrupt a splice acceptor, or to edit a regulatory sequence, thereby achieving correction of a pathogenic gene for therapeutic purposes.

### BRIEF DESCRIPTION OF THE DRAWINGS

FIG. 1 shows the editing efficiency of 005V1-nCas9 and 5V3354-nCas9 base editors at the PCSK9 target site.
FIG. 2 shows the editing efficiency of each mutant base editor at the PCSK9 target site.
FIG. 3 shows the base editing efficiency of 5V17.2-1249-nCas9, 5V22.1-1249-nCas9 and 5V22.2-1249-nCas9 at the PCSK9 gene target site (Position A6).

### DETAILED DESCRIPTION

### Definitions

The term "mutant" refers to a protein generated by mutation or recombinant DNA procedures.

The term "deaminase" refers to an enzyme that catalyzes a deamination reaction. The deaminase herein is a nucleobase deaminase, and the terms "deaminase" and "nucleobase deaminase" are used interchangeably herein. A deaminase may be a naturally occurring deaminase, or an active fragment or variant thereof. A deaminase may be active on a single-stranded nucleic acid such as ssDNA or ssRNA, or on a double-stranded nucleic acid such as dsDNA or dsRNA. In some embodiments, the deaminase only deaminates ssDNA and has no effect on dsDNA. In some embodiments, the deaminase is an adenosine deaminase or a cytidine deaminase.

The term "adenosine deaminase" or "adenosine deaminase protein" refers to a protein, a polypeptide, or one or more functional domains of a protein or polypeptide, capable of catalyzing the hydrolytic deamination reaction that converts adenine (or the adenine moiety of a molecule) to hypoxanthine (or the hypoxanthine moiety of a molecule). In some embodiments, the adenine-containing molecule is adenosine (A), and the hypoxanthine-containing molecule is inosine (I). The adenine-containing molecule may be deoxyribonucleic acid (DNA) or ribonucleic acid (RNA). Adenosine deaminases include, but are not limited to, members of the enzyme family known as Adenosine Deaminases Acting on RNA (ADAR), members of the enzyme family known as Adenosine Deaminases Acting on tRNA (ADAT), and other family members containing an adenosine deaminase domain (ADAD). According to the present disclosure, an adenosine deaminase is capable of targeting adenine in RNA/DNA and RNA duplexes. In particular embodiments, the adenosine deaminase has been modified to enhance its ability to edit DNA in RNA/DNA heteroduplexes of RNA duplexes.

The term "base editor" refers to a fusion protein comprising a nucleic acid-programmable nucleotide-binding protein (napDNAbp) (e.g., a nuclease) and a deaminase. "Base Editor (BE)" or "nucleobase editor" refers to an agent that binds a polynucleotide and has nucleobase-modifying activity. In various embodiments, a base editor comprises a nucleobase-modifying polypeptide (e.g., a deaminase) and a nucleic acid-programmable nucleotide-binding domain (e.g., a nucleic acid-programmable DNA-binding protein) that binds a guide polynucleotide (e.g., guide RNA). Examples of nucleic acid-programmable DNA-binding proteins include, but are not limited to, Cas9 (e.g., dCas9 and nCas9), CasX, CasY, Cpf1, C2c1, C2c2, C2c3, and Argonaute (AGO) proteins. In various embodiments, the agent is a biomolecular complex comprising a protein domain with base-editing activity, i.e., capable of modifying a base (e.g., A, T, C, G, or U) within a nucleic acid molecule (e.g., DNA, RNA). In some embodiments, the polynucleotide-programmable DNA-binding domain is fused or linked to a deaminase domain. In one embodiment, the agent is a fusion protein comprising a domain with base-editing activity. In some embodiments, the domain with base-editing activity is capable of deaminating a base within a nucleic acid molecule. In some embodiments, the base editor is capable of deaminating one or more bases within a DNA molecule. In some embodiments, the base editor is an Adenine Base Editor (ABE).

The term "nuclease" refers to an enzyme that catalyzes the cleavage of phosphodiester bonds between nucleotides in a nucleic acid molecule. In some embodiments, the DNA-binding polypeptide is an endonuclease capable of cleaving phosphodiester bonds between nucleotides within a nucleic acid molecule. In certain embodiments, the DNA-binding polypeptide is an exonuclease capable of cleaving nucleotides at either end (5' or 3') of a nucleic acid molecule. In some embodiments, the nuclease is selected from the group consisting of meganucleases, Zinc Finger Nucleases (ZFNs), Transcription Activator-Like Effector Nucleases (TALENs), RNA-guided nucleases, and homologs or variants thereof, wherein the nuclease activity is reduced or inhibited.

The term "meganuclease" refers to an endonuclease that binds a recognition site of 12 to 40 bp in length within dsDNA. Exemplary, non-limiting meganucleases include those of the LAGLIDADG family. "Meganuclease" may refer to a dimeric or single-chain meganuclease.

The term "Zinc Finger Nuclease (ZFN)" refers to a chimeric protein comprising a zinc finger DNA-binding domain and a nuclease domain.

The term "Transcription Activator-Like Effector Nuclease (TALEN)" refers to a chimeric protein comprising a TAL effector DNA-binding domain and a nuclease domain.

The term "nucleic acid-programmable DNA-binding protein" or "napDNAbp" is used interchangeably with "polynucleotide-programmable nucleotide-binding domain" and "nucleic acid-programmable nucleotide-binding domain", and means a protein associated with a nucleic acid (e.g., DNA or RNA), such that a guide nucleic acid or guide polynucleotide (e.g., gRNA) directs the napDNAbp to a specific nucleic acid sequence. In some embodiments, the polynucleotide-programmable nucleotide-binding domain is a polynucleotide-programmable DNA-binding domain. In some embodiments, the polynucleotide-programmable nucleotide-binding domain is a polynucleotide-programmable RNA-binding domain. In some embodiments, the nucleic acid-programmable nucleotide-binding protein is an RNA-guided nucleic acid-programmable nucleotide-binding protein. In some embodiments, the RNA-guided nucleic acid-programmable nucleotide-binding protein is an RNA-guided nuclease.

In some embodiments, said RNA-guided nuclease is selected from Type II CRISPR-Cas polypeptides, Type I CRISPR-Cas polypeptides, Type III CRISPR-Cas polypeptides, Type IV CRISPR-Cas polypeptides, Type V CRISPR-Cas polypeptides, Type VI CRISPR-Cas polypeptides, Type VII CRISPR-Cas polypeptides, IscB polypeptides, TnpB polypeptides, and IsrB polypeptides. In some embodiments, said polynucleotide-programmable nucleotide-binding domain is a Cas9 protein. A Cas9 protein may be associated with a guide RNA that directs the Cas9 protein to a specific DNA sequence complementary to the guide RNA. In some embodiments, the napDNAbp is a Cas9 domain, e.g., a nuclease-active Cas9, a Cas9 nickase (nCas9), or a nuclease-dead Cas9 (dCas9). Non-limiting examples of nucleic acid-programmable DNA-binding proteins include Cas9 (e.g., dCas9 and nCas9), Cas12a/Cpf1, Cas12b/C2c1, Cas12c/C2c3, Cas12d/CasY, Cas12e/CasX, Cas12g, Cas12h, Cas12i, Cas12j/CasΦ, Cas13a (C2c2), Cas13b, Cas13c, and Cas13d. Non-limiting examples of Cas enzymes include Cas1, Cas1B, Cas2, Cas3, Cas4, Cas5, Cas5d, Cas5t, Cas5h, Cas5a, Cas6, Cas7, Cas8, Cas8a, Cas8b, Cas8c, Cas9 (also known as Csn1 or Csx12), Cas10, Cas10d, Cas12a/Cpf1, Cas12b/C2c1, Cas12c/C2c3, Cas12d/CasY, Cas12e/CasX, Cas12g, Cas12h, Cas12i, Cas12j/CasΦ, Csy1, Csy2, Csy3, Csy4, Cse1, Cse2, Cse3, Cse4, Cse5e, Csc1, Csc2, Csa5, Csn1, Csn2, Csm1, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx1S, Csx11, Csf1, Csf2, CsO, Csf4, Csd1, Csd2, Cst1, Cst2, Csh1, Csh2, Csa1, Csa2, Csa3, Csa4, Csa5, Type II Cas effector proteins, Type V Cas effector proteins, Type VI Cas effector proteins, CARF, DinG, homologs thereof, or modified or engineered forms thereof. Other nucleic acid-programmable DNA-binding proteins are also within the scope of the present disclosure, even if not specifically listed herein. See, e.g., Makarova et al., "Classification and Nomenclature of CRISPR-Cas Systems: Wherefrom Here?" (CRISPR J. 2018 Oct; 1:325-336. doi:10.1089/crispr.2018.0033); Yan et al., "Functionally diverse type V CRISPR-Cas systems" (Science. 2019 Jan 4; 363(6422):88-91. doi:10.1126/science.aav7271), which describe Cas9 domains that bind non-canonical PAM sequences, the entire contents of which are incorporated herein by reference.

As used in the present disclosure, "base-editing activity" refers to the chemical alteration of a base within a polynucleotide. In one embodiment, a first base is converted to a second base. In one embodiment, said base-editing activity is adenosine or adenine deaminase activity, e.g., converting a target A•T base pair to a C•G base pair.

In some examples, base-editing activity is assessed by editing efficiency. Base-editing efficiency may be measured by any suitable means, e.g., by Sanger sequencing or next-generation sequencing (NGS). In some embodiments, base-editing efficiency is measured as the percentage of total sequencing reads bearing a nucleobase conversion affected by the base editor, e.g., the percentage of total sequencing reads bearing a target C•G base pair converted to an A•T base pair. In some embodiments, when base editing is performed in a cell population, base-editing efficiency is measured as the percentage of total cells bearing a nucleobase conversion affected by the base editor.

A "guide polynucleotide," "guide RNA," or "gRNA" refers to a polynucleotide that can specifically target a sequence of interest and can form a complex with a nucleic acid-programmable nucleotide-binding domain protein (e.g., Cas9). In one embodiment, the guide polynucleotide is a guide RNA (gRNA). A gRNA may exist as a complex of two or more RNAs, or as a single RNA molecule. A gRNA present as a single RNA molecule may be referred to as a single guide RNA (sgRNA), although "gRNA" is used interchangeably to refer to a guide RNA present as a single molecule or as a complex of two or more molecules. Typically, a gRNA present as a single RNA species comprises two domains: (1) a domain with homology to a target nucleic acid (e.g., that directs binding of a Cas9 complex to the target nucleic acid); and (2) a domain that binds a Cas9 protein. In some embodiments, domain (2) corresponds to a sequence known as a tracrRNA and includes stem-loop structures. For example, in some embodiments, domain (2) is identical or homologous to the tracrRNA provided in Jinek et al., Science 337:816-821 (2012). In some embodiments, a gRNA comprises two or more of domains (1) and (2), and may be referred to as an "extended gRNA." An extended gRNA will bind two or more Cas9 proteins and bind a target nucleic acid at two or more distinct regions. A gRNA comprises a nucleotide sequence complementary to a target site, which mediates binding of the nuclease/RNA complex to said target site, thereby providing sequence specificity to the nuclease:RNA complex.

The term "identity" refers to the degree of sequence matching between two polypeptides or two nucleic acids. "Identity" represents the percentage of identical residues between the polypeptide or nucleic acid sequences relative to the total number of residues, wherein the total number of residues is calculated based on the types of mutations. Types of mutations include insertions (extensions) at either or both ends of a sequence, deletions (truncations) at either or both ends of a sequence, substitutions/replacements of one or more amino acids/nucleotides, insertions within a sequence, and deletions within a sequence. Taking a polypeptide sequence as an example, if the type(s) of mutation is/are one or more of the following: substitutions/replacements of one or more amino acids/nucleotides, insertions within a sequence, and deletions within a sequence, the total number of residues is calculated based on the larger molecule in the comparison. If the types of mutations further include insertions (extensions) at either or both ends of a sequence or deletions (truncations) at either or both ends of a sequence, the number of amino acids inserted or deleted at either or both ends (e.g., less than 20 amino acids inserted or deleted at both ends) is not counted in the total number of residues. In calculating the percent identity, the sequences being compared are aligned in a manner that produces the maximal match between the sequences, and gaps in the alignment (if any) are resolved by specified algorithms. The calculation of nucleotide identity is analogous.

The terms "sequence identity" and "sequence homology" are used interchangeably herein, and, when used in connection with a polynucleotide or polypeptide, refer to the percentage of bases or amino acids that are identical and in the same relative positions when two sequences of a polypeptide or polynucleotide are compared or aligned. Sequence identity can be determined in a number of different ways. For example, sequences can be aligned using various methods and computer programs (e.g., BLAST, T-COFFEE, MUSCLE, MAFFT, etc.).

The term "DNA sequence or DNA polynucleotide sequence encoding a specific RNA" refers to a DNA sequence that can be transcribed into RNA. A DNA polynucleotide may encode an RNA that is translated into a protein (mRNA), or a DNA polynucleotide may encode an RNA that is not translated into a protein (e.g., tRNA, rRNA, or guide RNA; also referred to as "non-coding" RNA or "ncRNA"). A DNA sequence or DNA polynucleotide sequence may also "encode" a specific polypeptide or protein sequence, wherein, for example, the DNA directly encodes an mRNA that is translatable into a polypeptide or protein sequence. A "protein-coding sequence" or a sequence encoding a specific protein or polypeptide is a nucleic acid sequence that, when placed under the control of appropriate regulatory sequences, is capable of being transcribed into mRNA (in the case of DNA) and translated (in the case of mRNA) into a polypeptide *in vitro* or *in vivo*. The boundaries of the coding sequence are determined by a start codon at the 5'-terminus (N-terminus) and a translation termination nonsense codon at the 3'-terminus (C-terminus). A coding sequence may include, but is not limited to, cDNA from prokaryotic or eukaryotic mRNA, genomic DNA sequences from prokaryotic or eukaryotic DNA, and synthetic nucleic acids. Transcription termination sequences will generally be located at the 3'-end of the coding sequence.

The term "promoter" or "promoter sequence" refers to a DNA regulatory sequence capable of promoting transcription of an operably linked coding or non-coding sequence (e.g., a downstream (3' direction) coding or non-coding sequence), for example by binding RNA polymerase (e.g., capable of causing or increasing a detectable level of transcription relative to the level provided in the absence of said promoter). Various promoters, including inducible promoters and constitutive promoters, can be used to drive the vectors disclosed herein. A constitutive promoter is a nucleotide sequence that, when operably linked to a polynucleotide encoding or defining a gene product, results in production of the gene product in a cell under most or all physiological conditions of the cell. Examples of promoters known in the art that can be used in certain embodiments (e.g., in the viral vectors disclosed herein) include the CMV promoter, CBA promoter, smCBA promoter, and promoters derived from immunoglobulin genes, SV40, or other tissue-specific genes (e.g., RLBP1, RPE, VMD2). Moreover, standard techniques for generating functional promoters by mixing and matching known regulatory elements are known in the art. Fragments of promoters can also be used, such as those retaining at least a minimal number of bases or elements to initiate detectable levels of transcription above background.

"Operably linked" refers to a linkage between genetic elements such that a target nucleotide sequence is linked to regulatory elements in a manner permitting expression of the nucleotide sequence (e.g., in an *in vitro* transcription/translation system or in a host cell when the vector is introduced into the host cell). For example, a promoter needs to be positioned upstream of the coding sequence of the gene it controls in order to initiate transcription of the gene. The promoter must be properly placed upstream of the coding sequence with an appropriate linkage therebetween, so that the promoter can effectively initiate transcription of the coding sequence. Advantageous vectors include lentiviruses and adeno-associated viruses, and the types of these vectors can also be selected to target specific cell types.

The term "vector" refers to a nucleic acid molecule capable of transporting another nucleic acid molecule to which it is linked. Vectors include, but are not limited to, single-stranded, double-stranded, or partially double-stranded nucleic acid molecules; nucleic acid molecules with one or more free ends or no free ends (e.g., circular); nucleic acid molecules comprising DNA, RNA, or both; and other diverse polynucleotides known in the art. A vector can be introduced into a host cell via transformation, transduction, or transfection, such that the genetic material elements it carries are expressed in the host cell. A vector can be introduced into a host cell to thereby produce transcripts, proteins, or peptides, including proteins, fusion proteins, isolated nucleic acid molecules, etc., as described herein (e.g., CRISPR transcripts, such as nucleic acid transcripts, proteins, or enzymes). A vector may contain a variety of expression control elements, including but not limited to promoter sequences, transcription initiation sequences, enhancer sequences, selection markers, and reporter genes. A vector may also contain an origin of replication. Vectors include plasmids and viral vectors. A plasmid refers to a circular double-stranded DNA loop into which additional DNA fragments can be inserted, e.g., by standard molecular cloning techniques. Viral vectors are those in which viral-derived DNA or RNA sequences are present in a vector for packaging of a virus; viruses include, e.g., retroviruses, replication-defective retroviruses, adenoviruses, replication-defective adenoviruses, and adeno-associated viruses. Viral vectors also include polynucleotides carried by a virus for transfection into a host cell. Some vectors (e.g., bacterial vectors with a bacterial origin of replication and episomal mammalian vectors) are capable of autonomous replication in a host cell into which they are introduced. Other vectors (e.g., non-episomal mammalian vectors) integrate into the genome of a host cell upon introduction, and thereby replicate along with the host genome. Moreover, certain vectors are capable of directing the expression of genes to which they are operably linked. Such vectors are referred to as "expression vectors".

The term "wild-type" has the meaning commonly understood by a person skilled in the art, referring to the typical form of an organism, strain, gene, or protein, or the characteristics distinguishing it from mutant or variant forms when it exists in nature, which can be isolated from natural sources without intentional artificial modification.

The terms "variant", "derivative" and "analog" refer to a polypeptide that substantially retains the function or activity of a protein. Generally, derivatization of a protein does not adversely affect the desired activity of the protein, i.e., a derivative of a protein has the same activity as the protein. Modified forms of a "derivative" include those in which one or more amino acids of the protein may be deleted, inserted, modified and/or substituted.

The terms "non-naturally occurring" and "engineered" are used interchangeably and indicate the involvement of human intervention.

As used herein, a "functional fragment" or "active fragment" of a polynucleotide or polypeptide may refer to any subset of contiguous nucleotides or contiguous amino acids that retains the original (e.g., wild-type) activity (or substantially similar activity) of the polynucleotide or polypeptide, respectively. In some examples, the "functional fragment" or "active fragment" comprises any part or subsequence of the original (e.g., wild-type) or mutant polynucleotide or polypeptide. In some embodiments, the activity of the "functional fragment" or "active fragment" of the polynucleotide or polypeptide may, illustratively, be about 100%, 99%, 98%, 97%, 96%, 95%, 94%, 93%, 92%, 91%, 90%, 85%, 80%, 75%, 70%, 65%, 60%, 55%, 50%, 45%, 40%, 35%, 30%, 25%, 20%, 15%, 10%, or less than 10% of the activity relative to the original (e.g., wild-type) activity.

Nucleic acid cleavage in the present disclosure includes: DNA or RNA cleavage in a target nucleic acid generated by the Cas protein (cis cleavage), and cleavage of DNA or RNA in collateral nucleic acid substrates (single-stranded nucleic acid substrates) caused by the collateral cleavage activity of the Cas protein (i.e., non-specific or non-targeted, trans cleavage). In some embodiments, the cleavage is a double-stranded DNA break. In some embodiments, the cleavage is a single-stranded DNA break or a single-stranded RNA break.

The term "Clustered Regularly Interspaced Short Palindromic Repeats (CRISPR)-CRISPR-associated (Cas) system" or "CRISPR-Cas system" are used interchangeably and have the meaning commonly understood by a person skilled in the art, generally comprising transcripts or other elements related to the expression of CRISPR-associated ("Cas") genes, or transcripts or other elements capable of directing the activity of said Cas genes.

The terms "target nucleic acid" and "target sequence" are used interchangeably and refer to a specific nucleic acid comprising a nucleic acid sequence fully or partially complementary to the guide sequence in a gRNA. A "target sequence" refers to a polynucleotide targeted by the guide sequence in a gRNA, e.g., a sequence complementary to the guide sequence, wherein hybridization between the target sequence and the guide sequence facilitates formation of a CRISPR/Cas complex comprising a Cas protein and a gRNA. Perfect complementarity is not required, so long as sufficient complementarity exists to permit hybridization and promote formation of a CRISPR/Cas complex. In some examples, the target nucleic acid comprises a non-coding region (e.g., a promoter or terminator). In some examples, the target nucleic acid is single-stranded or double-stranded. The target sequence may comprise any polynucleotide, such as DNA or RNA. In certain cases, the target sequence is located intracellularly or extracellularly. In certain cases, the target sequence is located in the nucleus, cytoplasm, or organelle (e.g., mitochondria or chloroplasts) of a cell. The target nucleic acid may be a sequence encoding a gene product (e.g., a protein) or a non-coding sequence (e.g., a regulatory polynucleotide or junk DNA). In certain cases, the target sequence is associated with a protospacer adjacent motif (PAM).

The detection method of the present disclosure can be used for quantitative detection of a target nucleic acid to be detected. The quantitative detection index can be quantified according to the signal intensity of a reporter group, such as the luminescence intensity of a fluorescent group, or the width of a chromogenic band.

The term "regulatory element" includes promoters, enhancers, internal ribosome entry sites (IRES), and other expression control elements (e.g., transcription termination signals, such as polyadenylation signals and poly-U sequences). In some cases, regulatory elements include those directing constitutive expression of a nucleotide sequence in many types of host cells and those directing expression of the nucleotide sequence only in certain host cells (e.g., tissue-specific regulatory sequences). A tissue-specific promoter may direct expression primarily in a desired tissue of interest, such as muscle, neuron, bone, skin, blood, a specific organ (e.g., liver, pancreas), or a specific cell type (e.g., lymphocyte). In other cases, regulatory elements may also direct expression in a temporal-dependent manner (e.g., in a cell cycle-dependent or developmental stage-dependent manner), which may or may not be tissue or cell type-specific.

The term "host cell" refers to a eukaryotic cell (e.g., animal cell, plant cell, fungal cell, etc.), a prokaryotic cell (e.g., certain microbial cells, *Escherichia coli*, *Bacillus subtilis*, etc.), or a cell from a multicellular organism (e.g., a cell line) cultured as a unicellular entity, which serves as a recipient for a nucleic acid (e.g., an expression vector), and includes the progeny of the original cell genetically modified with the nucleic acid.

It is understood that the progeny of a single cell may not necessarily be identical in morphology, genome, etc., to the original parent cell due to natural, incidental, or intentional mutations. A "recombinant host cell" (also referred to as a "genetically modified host cell") is a host cell into which a heterologous nucleic acid, e.g., an expression vector, has been introduced.

A person skilled in the art will appreciate that the design of an expression vector may depend on factors such as the choice of host cell to be transformed, the desired level of expression, and the like.

The term "NLS" refers to a "nuclear localization sequence" or "nuclear localization signal", meaning an amino acid sequence that directs a protein into the cell nucleus. Nuclear localization sequences are known in the art (e.g., as described in International PCT Application PCT/EP2000/011690, filed Nov 23, 2000 and published as WO/2001/038547 on May 31, 2001), which is incorporated herein by reference for its disclosure of exemplary nuclear localization sequences. In other embodiments, the NLS is an optimized NLS, e.g., as described in Koblan et al., Nature Biotech. 2018, doi:10.1038/nbt.4172. In some examples, the NLS comprises any one of the following amino acid sequences: KRTADGSEFESPKKKRKV (SEQ ID NO: 24), AVKRPAATKKAGQAKKKKLD (SEQ ID NO: 25), KRPAATKKAGQAKKKK (SEQ ID NO: 26), KKTELQTTNAENKTKKL (SEQ ID NO: 27), KRGINDRNFWRGENGRKTR (SEQ ID NO: 28), RKSGKIAAIVVKRPRK (SEQ ID NO: 29), PKKKRKV (SEQ ID NO: 30), or MDSLLMNRRKFLYQFKNVRWAKGRRETYLC (SEQ ID NO: 31).

The term "complementarity" refers to the ability of one nucleic acid sequence to form one or more hydrogen bonds with another nucleic acid sequence via conventional Watson-Crick base pairing or other non-conventional types. Percent complementarity refers to the percentage of residues in one nucleic acid molecule that can form hydrogen bonds (e.g., Watson-Crick base pairing) with another nucleic acid sequence (e.g., 5, 6, 7, 8, 9, 10 out of 10 complementary residues correspond to 50%, 60%, 70%, 80%, 90%, and 100% complementarity, respectively). "Perfectly complementary" means that all contiguous residues of one nucleic acid sequence form hydrogen bonds with the same number of contiguous residues in another nucleic acid sequence. "Substantially complementary" refers to a degree of complementarity of at least 60%, 65%, 70%, 75%, 80%, 85%, 90%, 95%, 97%, 98%, 99%, or 100% over a region of 8, 9, 10, 11, 12, 13, 14, 15, 16, 17, 18, 19, 20, 21, 22, 23, 24, 25, 30, 35, 40, 45, 50 or more nucleotides, or refers to two nucleic acids that hybridize under stringent conditions.

Hybridization between a target sequence and a gRNA means that the nucleic acid sequences of the target sequence and the gRNA are at least 60%, 65%, 70%, 75%, 80%, 85%, 90%, 91%, 92%, 93%, 94%, 95%, 96%, 97%, 98%, 99%, or 100% complementary and capable of hybridizing to form a complex; or means that at least 12, 15, 16, 17, 18, 19, 20 or more bases in the nucleic acid sequences of the target sequence and the gRNA are capable of complementary pairing to hybridize and form a complex.

The term "delivery" refers to providing an entity (e.g., a drug) to a destination. For example, components of the CRISPR-Cas system/compositions of the present disclosure can be delivered in various forms, such as DNA/RNA, RNA/RNA, or protein-RNA combinations. For instance, a Cas protein may be delivered as a polynucleotide encoding DNA, a polynucleotide encoding RNA, or as a protein.

The term "linker" refers to a linear polypeptide formed by linking multiple amino acid residues via peptide bonds. A synthetic amino acid sequence or a naturally occurring polypeptide sequence may be chosen as the linker.

The term "effective amount" or "therapeutically effective amount" refers to a dose sufficient to achieve a beneficial or desired result. A therapeutically effective amount may depend on the individual being treated and the disease condition, the weight and age of the individual, the severity of the disease condition, the mode of administration, and the like, which can be readily determined by a person skilled in the art.

The terms "treatment", "treating" and the like refer to obtaining a desired pharmacological and/or physiological effect, e.g., treating or curing a condition in a subject, delaying the onset of symptoms of the condition and/or delaying the severity of the condition. The effect may be prophylactic in terms of complete or partial prevention of the disease or its symptoms, and/or therapeutic in terms of partial or complete cure of the disease and/or side effects attributable to the disease. As used herein, "treatment" encompasses any treatment of a disease in a mammal (e.g., a human) and includes: (a) preventing the occurrence of a disease in a subject who may be predisposed to the disease but has not yet been diagnosed as having it; (b) inhibiting the disease, i.e., arresting its development; and (c) relieving the disease, i.e., causing regression of the disease.

The terms "individual", "subject", "host" and "patient" refer to an individual organism, including but not limited to various animals, plants, and microorganisms. Animals include mammals, including but not limited to bovines, equines, ovines, porcines, canines, felines, lagomorphs, rodents (e.g., mice or rats), apes, non-human primates (e.g., rhesus monkeys or cynomolgus monkeys), humans, mammalian farm animals, mammalian sport animals, and mammalian pets. In certain embodiments, the subject (e.g., a human) has a condition (e.g., a condition caused by a disease-associated genetic defect). A "plant" is any differentiated multicellular organism capable of photosynthesis, including crop plants at any stage of maturity or development.

It should be understood that any embodiment of the present disclosure described herein, including those described only in the Examples or Claims, or only in one aspect/section below, may be combined with any one or more other embodiments of the present disclosure unless explicitly disclaimed or inappropriate for combination.

The present disclosure is further illustrated by the following examples, which are not intended to limit the scope of the present disclosure to the scope of the described examples.

Where no specific conditions are indicated in the Examples, the procedures are performed under conventional conditions or conditions recommended by the manufacturer. Reagents or instruments for which the manufacturer is not indicated are conventional products commercially available. A person skilled in the art will recognize that the Examples describe the present disclosure by way of illustration and are not intended to limit the scope of protection claimed by the present disclosure. All publications and other references mentioned herein are incorporated by reference in their entirety.

### Example 1: Obtainment of Deaminase Mutants

### (1) Obtainment of Deaminase Mutant 5V3354

To construct an adenosine deaminase with higher editing efficiency and specificity, functional prediction of amino acid sites was performed on the amino acid sequence of the known adenosine deaminase 005V1 (deaminase 005V1 of CN114634923A, whose amino acid sequence is shown in SEQ ID NO: 1 and nucleotide coding sequence is shown in SEQ ID NO: 2), and multiple positions that may improve editing efficiency and specificity were identified. Expression vectors comprising an adenosine deaminase 005V1 variant-nCas9 were constructed by site-directed PCR mutagenesis of the expression vector of base editor 005V1-nCas9 containing wild-type deaminase 005V1 (the amino acid sequence of base editor 005V1-nCas9 is shown in SEQ ID NO: 3, and the nucleotide coding sequence is shown in SEQ ID NO: 4).

Base editors of different deaminase variants were generated by PCR-based site-directed mutagenesis. Specifically, the DNA sequence encoding base editor 005V1-nCas9 (SEQ ID NO: 4) was amplified with multiple amino acids near the mutation site as the center, and sequences to be mutated were introduced into the primers. Different mutant base editors were obtained by homologous recombination ligation of the amplified fragments (Table 1), and mutant 5V3354 was acquired.

**Table 1. Mutation mode of deaminase mutant 5V3354**

| Mutant | Amino acid mutation mode and codons after mutation |
|---|---|
| 5V3354 | A46C+I47Y+T48G+L49H+V104M |
| | TGC+TAC+GGC+CAT+ATG |

The specific mutation procedure is as follows:
The plasmid expressing the 005V1-nCas9 base editor was used as the template. Amplification of the plasmid encoding the 005V1-nCas9 base editor was performed using a high-fidelity PCR kit (Vazyme, P501-d2) with amplification primers containing the mutation sequences.

The amplification reaction system is shown in Table 2:

**Table 2. Plasmid mutation amplification system for 005V1-nCas9 base editor**

| Components | 100 µL system |
|---|---|
| 2 × Phanta Flash Master Mix (Dye Plus) | 50 µL |
| 10 µM Forward Primer | 5 µL |
| 10 µM Reverse Primer | 5 µL |
| Plasmid DNA | 5 ng |
| Nuclease-Free Water | to 100 µL |

The PCR amplification program is shown in the table below:

**Table 3. PCR program for plasmid mutation amplification of 005V1-nCas9 base editor**

| Step | TEMP | Time |
|---|---|---|
| Initial Denaturation | 95°C | 30s |
| 34 Cycles | 95°C | 10s |
| | 60°C | 30s |
| | 72°C | 40s |
| Final Extension | 72°C | 2min |
| Hold | 4°C | forever |

The amplified PCR product was recovered and purified using a universal DNA purification kit (TIANGEN, DP214). The purified PCR product was transformed into *Escherichia coli* DH5α competent cells (Weidi Biotechnology, DL1001) and cultured. Single colonies were picked and verified by sequencing. Positive clones were incubated with shaking, and plasmids were extracted using an Endo-Free Plasmid Maxiprep Kit (TIANGEN, DP120-01). The plasmids were stored at -20°C for subsequent use.

### (2) Validation of Editing Activity of Deaminase Mutant 5V3354

To test the editing activity of deaminase mutant 5V3354, the PCSK9 target sequence PCSK9-sgRNA: cccgcaccttggcgcagcgg (SEQ ID NO: 5) was designed for the PCSK9 gene.

The construction process of the sgRNA expression vector (sgRNA plasmid) is as follows:
sgRNAs were designed according to the target sequence, and oligonucleotides (oligos) were synthesized. The sgRNA targeting sequence used is shown in SEQ ID NO: 5. The sequence CACC was added to the 5'-end of the upstream sgRNA targeting sequence, and the sequence AAAC was added to the 5'-end of the downstream sequence. Accordingly, the upstream and downstream primer sequences for synthesis were PCSK9-sgRNA-F (SEQ ID NO: 6) and PCSK9-sgRNA-R (SEQ ID NO: 7), respectively.

After synthesis, the upstream and downstream sequences were annealed using a preset PCR program (95°C for 5 min; 95°C to 85°C at -2°C/s; 85°C to 25°C at -0.1°C/s; hold at 4°C). The annealed product was ligated into the lenti U6-sgRNA/EF1a-mCherry vector (Addgene, Plasmid #114199) linearized with BbsI (NEB, R3539S).

The reaction systems used for sgRNA plasmid construction are as follows:
Linearization system for the lenti U6-sgRNA/EF1a-mCherry vector: 3 µg vector; 6 µL buffer (NEB: R0539L); 2 µL BbsI; supplemented with ddH2O to 60 µL, digested overnight at 37°C.

Ligation system for sgRNA annealed product and linearized vector: 1 µL T4 ligase buffer (NEB, M0202L), 20 ng linearized vector, 5 µL annealed oligo fragment (10 µM), 0.5 µL T4 ligase (NEB: M0202L), supplemented with ddH2O to 10 µL, ligated overnight at 16°C.

The ligated vector was transformed into *Escherichia coli* DH5α competent cells (Weidi Biotechnology, DL1001). The specific procedure is as follows: DH5α competent cells were taken out from -80°C and immediately inserted into ice; after the cell pellet melted for 5 minutes, the ligation product was added and mixed gently by tapping the bottom of the centrifuge tube, followed by incubation on ice for 25 minutes. Heat shock was performed in a 42°C water bath for 45 seconds, and the tube was immediately returned to ice and incubated for 2 minutes. 700 µL antibiotic-free sterile LB medium was added to the centrifuge tube, mixed well, and recovered at 37°C, 200 rpm for 60 minutes. The cells were collected by centrifugation at 5000 rpm for 1 minute, approximately 100 µL supernatant was retained to gently resuspend the cell pellet, which was then spread on LB medium supplemented with ampicillin. The plate was inverted and cultured overnight in a 37°C incubator. Single colonies were picked and verified by sequencing; positive clones were cultured with shaking, and sgRNA plasmids were extracted using an Endo-Free Plasmid Maxiprep Kit (TIANGEN, DP120-01). The plasmid concentration was determined, and the plasmids were stored at -20°C for subsequent use.

HEK293T cells (purchased from ATCC) were seeded in DMEM medium (Gibco, 11965092) supplemented with 10% (v/v) fetal bovine serum (FBS) and 1% (v/v) Penicillin-Streptomycin (Gibco, 15140122), and cultured in a 37°C cell incubator with 5% CO₂. Cells for transfection were seeded in 24-well cell culture plates one day in advance; transfection was performed the next day when the cell density reached approximately 80%. The transfection dosage per well of the 24-well plate was 0.4 µg editor fusion protein plasmid and 0.4 µg sgRNA plasmid.

The mixed plasmids were diluted with 25 µL serum-reduced medium (Yuanpei Biotechnology, L530KJ), followed by addition of 2 µL P3000 reagent, mixed by pipetting to prepare Reagent A, and incubated for 5 minutes. Meanwhile, 2 µL Lipofectamine 3000 transfection reagent (Thermo, 11668019) was diluted with 25 µL serum-reduced medium and mixed to prepare Reagent B, which was incubated for 5 minutes. Reagent A and Reagent B were mixed, homogenized by pipetting, and incubated for 20 minutes. After incubation, the mixed reagent was added dropwise to the cells in the 24-well plate to be transfected, and the plate was returned to the 37°C incubator for culture. The medium was replaced with DMEM medium containing 10% FBS 6 hours after transfection. Cells were harvested 48 hours after transfection for editing efficiency detection.

Genomic DNA was extracted from the collected cells (TIANGEN, DP304-03). Primers were designed according to experimental requirements, and the identification primers used were PCSK9-F (SEQ ID NO: 8) and PCSK9-R (SEQ ID NO: 9).

PCR amplification of the sequence near the target site was performed using the genomic DNA as template. The system for target site sequence amplification is as follows: 2 × Taq Master Mix (Vazyme, P112-03): 25 µL; Primer-F (10 pmol/µL): 1 µL; Primer-R (10 pmol/µL): 1 µL; Template: 1 µL; ddH₂O: supplemented to 50 µL.

The amplified PCR products were subjected to high-throughput deep sequencing (Genewiz Biotechnology Co., Ltd.) or Sanger sequencing (Boshang Biotechnology (Shanghai) Co., Ltd.) for identification of editing efficiency.

Detection of gene editing efficiency:
The calculation method for gene editing efficiency was performed according to Kluesner MG, Nedveck DA, Lahr WS, Garbe JR, Abrahante JE, Webber BR, Moriarity BS. EditR: A Method to Quantify Base Editing from Sanger Sequencing. CRISPR J. 2018 Jun;1(3):239-250. doi:10.1089/crispr.2018.0014. PMID: 31021262; PMCID: PMC6694769.

HEK293T cells were co-transfected with the 005V1-nCas9 base editor plasmid and the sgRNA plasmid in the same manner, and the editing efficiency was calculated.

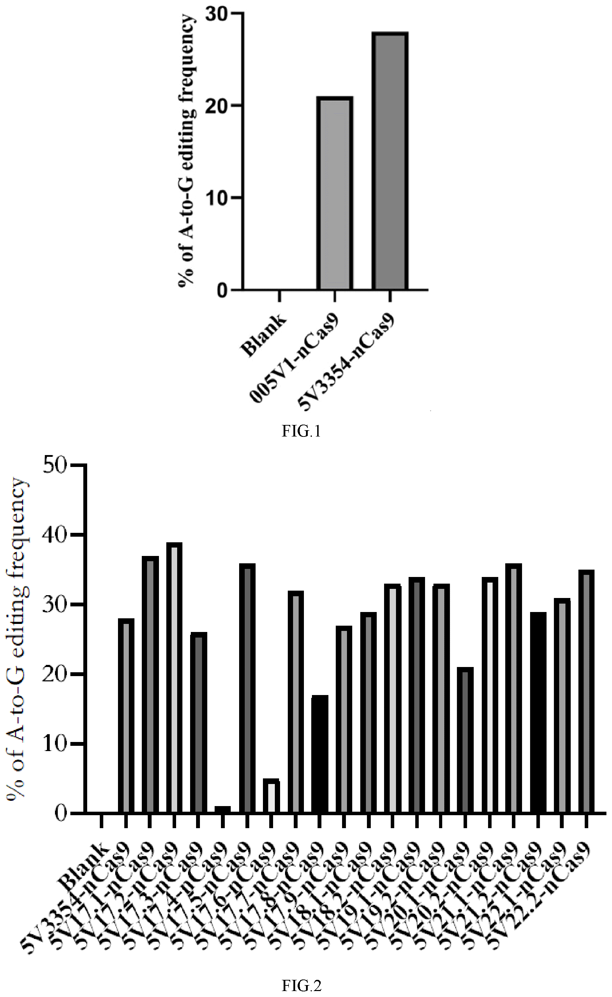

In this example, the structure of the base editor comprising the adenosine deaminase provided by the present disclosure and nCas9 is as follows:
NH₂-[NLS]-[adenosine deaminase]-linker-[nCas9]-[NLS]-COOH. An exemplary amino acid sequence of this structure is shown in SEQ ID NO: 3, which is for illustrative purposes only and is not intended to limit the structure of the base editor. This example quantified the editing efficiency of the 005V1-nCas9 and 5V3354-nCas9 base editors at the PCSK9 target site (site A6, efficiency of adenine A to guanine G mutation). Among them, 005V1-nCas9 exhibited a base editing efficiency of 21% at this site, whereas 5V3354-nCas9 achieved a base editing efficiency of 28% at this site (FIG. 1).

### Example 2: Obtainment of Other Deaminase Mutants

(1)To obtain base editors with higher editing efficiency, further mutagenesis was performed based on deaminase 5V3354 (amino acid sequence shown in SEQ ID NO: 10, nucleotide coding sequence shown in SEQ ID NO: 11). Using the same method as Example 1, PCR mutagenesis was conducted on the expression vector of deaminase 5V3354-nCas9 to obtain a variety of deaminase mutants (Table 4).

**Table 4. Mutation modes of each mutant of deaminase 5V3354**

| Mutants | Amino acid mutation mode and codons after mutation |
|---|---|
| 5V17.1 | Q148R+Q149N+P150A+E152P+V153T+F154S+N155P |
| | CGG+AAT+GCT+CCT+ACC+TCC+CCC |
| 5V17.2 | Q148R+P150L+E152L+V153A+F154P+N155G |
| | CGA+TTG+CTC+GCC+CCC+GGG |
| 5V17.3 | Q148R+Q149R+P150R+E152P+V153F+F154V+N155T |
| | AGA+CGT+CGA+CCA+TTC+GTC+ACA |
| 5V17.4 | Q148G+Q149G+P150I+E152L+V153Y+F154N+N155S |
| | GGT+GGT+ATC+TTA+TAC+AAC+TCG |
| 5V17.5 | Q149P+P150G+R151K+E152Q+V153K+F154L+N155P |
| | CCT+GGA+AAG+CAG+AAG+CTC+CCG |
| 5V17.6 | Q148R+Q149V+P150S+R151P+E152L+V153F+F154P+N155Y |
| | CGC+GTC+TCT+CCG+TTA+TTC+CCC+TAT |
| 5V17.7 | Q148T+Q149G+P150R+E152H+V153A+F154D+N155S |
| | ACT+GGT+CGT+CAC+GCT+GAT+TCT |
| 5V17.8 | Q148S+Q149F+P150L+E152S+V153P+F154L+N155R |
| | TCG+TTT+TTG+TCA+CCT+CTG+CGC |
| 5V17.9 | Q148G+Q149C+P150S+E152P+F154H+N155A |
| | GGA+TGT+TCT+CCA+CAC+GCC |
| 5V18.1 | A156L+E157F+R158N+E159L+A160K+R161K+R162L+L163D |
| | TTA+TTT+AAT+CTT+AAA+AAA+CTG+GAT |
| 5V18.2 | A156T+E157F+R158L+E159H+A160T+R162K+L163I |
| | ACA+TTC+CTC+CAC+ACG+AAA+ATT |
| 5V19.1 | N164G+Q165T+P166Q+D167L+R168L+A169N+D170R |
| | GGT+ACT+CAG+CTT+TTA+AAC+CGG |
| 5V19.2 | N164R+Q165L+D167L+R168P+A169T+D170H |
| | CGC+CTA+CTC+CCG+ACA+CAT |
| 5V20.1 | C144T+Q145L+F146A+Y147S+Q148R+Q149K+P150S |
| | ACT+CTC+GCG+TCT+CGA+AAA+TCA |
| 5V20.2 | C144L+Q145L+F146R+Y147F+Q148C+P150T |
| | CTT+CTA+CGT+TTT+TGC+ACA |
| 5V21.1 | C144W+Q145K+Q148R+Q149R+P150R+E152P+V153F+F154V+N155T |
| | TGG +AAG +AGA+CGT+CGA+CCA+TTC+GTC+ACA |
| 5V21.2 | C144W+Q145K |
| | TGG+AAG |
| 5V22.1 | G48A+Q148R+P150L+E152L+V153A+F154P+N155G |
| | GCC+CGA+TTG+CTC+GCC+CCC+GGG |
| 5V22.2 | C46P+Y47I+G48T+H49R+Q148R+P150L+E152L+V153A+F154P+N155G |
| | CCC+ATC+ACC+CGG+CGA+TTG+CTC+GCC+CCC+GGG |

The following base editors were obtained: 5V17.1-nCas9, 5V17.2-nCas9, 5V17.3-nCas9, 5V17.4-nCas9, 5V17.5-nCas9, 5V17.6-nCas9, 5V17.7-nCas9, 5V17.8-nCas9, 5V17.9-nCas9, 5V18.1-nCas9, 5V18.2-nCas9, 5V19.1-nCas9, 5V19.2-nCas9, 5V20.1-nCas9, 5V20.2-nCas9, 5V21.1-nCas9, 5V21.2-nCas9, 5V22.1-nCas9, 5V22.2-nCas9.

(2) The PCSK9-sgRNA expression plasmid was constructed in the same manner as Example 1. The expression plasmid of each obtained mutant base editor and the PCSK9-sgRNA expression plasmid were co-transfected into HEK293T cells, and the editing efficiency was detected (FIG. 2).

The comparison shows that, compared with the base editor constructed by wild-type 5V3354 (editing efficiency: 28%), the base editors constructed by mutants 5V17.1, 5V17.2, 5V17.5, 5V17.7, 5V18.1, 5V18.2, 5V19.1, 5V19.2, 5V20.2, 5V21.1, 5V21.2, 5V22.1 and 5V22.2 exhibit significantly improved editing efficiency at the PCSK9 target sequence, which are 37%, 39%, 36%, 32%, 29%, 33%, 34%, 33%, 34%, 36%, 29%, 31% and 35%, respectively. In particular, 5V17.2-nCas9 shows a base editing efficiency close to 40%.

In addition, the editing efficiency of the base editor constructed by 5V17.3 reaches 26%, and that of 5V17.9 reaches 27%, both of which are significantly superior to 005V1; the editing efficiency of 5V20.1 reaches 21%, which is comparable to that of 005V1.

### Example 3: Determination of Editing Efficiency of Chimeric Base Editors of Partial Mutants

To further investigate the activity of the mutants, chimeric recombinant base editors were designed for deaminase mutants 5V17.2, 5V22.1 and 5V22.2, in which the deaminase was inserted between residues 1249 and 1250 of nCas9, named 5V17.2-1249-nCas9, 5V22.1-1249-nCas9 and 5V22.2-1249-nCas9, respectively. The structure of the base editor is shown as follows: NH₂-[NLS]-[N-terminal fragment of nCas9]-[adenosine deaminase]-[C-terminal fragment of nCas9]-[NLS]-COOH.

The experimental procedures are as follows:

### 1. Construction of nCas9 Plasmid

(1) Cloning primers for nCas9 were designed (primers were synthesized by Boshang Biotechnology (Shanghai) Co., Ltd.), with the upstream and downstream primers being nCas9-F (SEQ ID NO: 12) and nCas9-R (SEQ ID NO: 13), respectively.

PCR amplification of ABE8e (Addgene, #138489) was performed using a high-fidelity PCR kit (Vazyme, P501-d2). The amplification system is shown in Table 5:

**Table 5. PCR amplification system for ABE8e (Addgene, #138489)**

| Components | 100 µL system |
|---|---|
| 2 × Phanta Flash Master Mix (Dye Plus) | 50 µL |
| 10 µM Forward Primer | 5 µL |
| 10 µM Reverse Primer | 5 µL |
| DNA | Plasmid(5ng) |
| Nuclease-Free Water | to 100 µL |

The PCR amplification program is shown in the table below:

**Table 6. PCR amplification program for ABE8e (Addgene, #138489)**

| Step | TEMP | Time |
|---|---|---|
| Initial Denaturation | 95°C | 30s |
| 34 Cycles | 95°C | 10s |
| | 60°C | 30s |
| | 72°C | 40s |
| Final Extension | 72°C | 2min |
| Hold | 4°C | forever |

The amplified PCR product was recovered according to the kit instructions (TIANGEN, Universal DNA Purification Kit, DP214). The purified PCR product was transformed into *Escherichia coli* DH5α competent cells (Weidi Biotechnology, DL1001). The specific procedure is as follows:
DH5α competent cells were taken out from -80°C and immediately placed on ice. After the cell pellet thawed for 5 minutes, the ligation product was added and mixed gently by tapping the bottom of the centrifuge tube, followed by incubation on ice for 25 minutes. Heat shock was performed in a 42°C water bath for 45 seconds, and the tube was immediately returned to ice and incubated for 2 minutes. 700 µL of antibiotic-free sterile LB medium was added to the centrifuge tube, mixed well, and the cells were recovered at 37°C, 200 rpm for 60 minutes. The cells were collected by centrifugation at 5000 rpm for 1 minute. Approximately 100 µL of the supernatant was retained to gently resuspend the cell pellet, which was then spread on LB medium supplemented with ampicillin. The plate was inverted and cultured overnight in a 37°C incubator. Single colonies were picked and verified by sequencing. Positive clones were cultured with shaking, and the nCas9 plasmid was extracted using an Endo-Free Plasmid Maxiprep Kit (TIANGEN: DP120-01). The plasmid concentration was determined, and the plasmid was stored at -20°C for subsequent use.

### (2) Obtainment of DNA Sequences Encoding Deaminases 5V17.2, 5V22.1 and 5V22.2

PCR amplification of the plasmids 5V17.2-nCas9, 5V22.1-nCas9 and 5V22.2-nCas9 was performed using primer pairs, with the amplification system and PCR program identical to those in Tables 5 and 6. The amplified PCR products were recovered according to the kit instructions (TIANGEN, Universal DNA Purification Kit, DP214), yielding PCR products of adenosine deaminases 5V17.2, 5V22.1 and 5V22.2. The upstream and downstream PCR primers used were ADA-F (SEQ ID NO: 14) and ADA-R (SEQ ID NO: 15).

### (3) Design of Different Chimeric Base Editors and Corresponding Primer Sequence Design for nCas9 Plasmids

Primer sequences corresponding to nCas9 were designed based on the insertion site of the deaminase, as detailed below:
nCas9-1249-F: ggggcagcagcggggggtcacccgaggataatgagcagaaacagctgt (SEQ ID NO: 16)
nCas9-1249-R: CCGCCGCTAGATCCTCCAGAggagcccttcagcttctcatagtggct (SEQ ID NO: 17)

The nCas9 plasmid obtained in step (1) was amplified separately using the above primer sequences, with the amplification system and PCR program identical to those in Tables 5 and 6. The amplified PCR products were recovered according to the kit instructions (TIANGEN, Universal DNA Purification Kit, DP214). The amplified nCas9 PCR products were subjected to homologous recombination with the PCR products of adenosine deaminases 5V17.2, 5V22.1 and 5V22.2 obtained in step (2), respectively, to construct chimeric base editors 5V17.2-1249-nCas9, 5V22.1-1249-nCas9 and 5V22.2-1249-nCas9 (Table 7). The kit used for homologous recombination was Gibson Assembly Master Mix (NEB, E2611S).

**Table 7. Chimeric base editors 5V17.2-1249-nCas9, 5V22.1-1249-nCas9 and 5V22.2-1249-nCas9**

| Base Editor | Corresponding Amino Acid Sequence | Nucleotide Coding Sequence |
|---|---|---|
| 5V17.2-1249-nCas9 | SEQ ID NO: 18 | SEQ ID NO: 19 |
| 5V22.1-1249-nCas9 | SEQ ID NO: 20 | SEQ ID NO: 21 |
| 5V22.2-1249-nCas9 | SEQ ID NO: 22 | SEQ ID NO: 23 |

### (4) Amplification and Sequencing of Chimeric Base Editors 5V17.2-1249-nCas9, 5V22.1-1249-nCas9 and 5V22.2-1249-nCas9

The homologous recombination products were transformed into *Escherichia coli* DH5α competent cells (Weidi Biotechnology, DL1001). The specific procedure is as follows: DH5α competent cells were taken out from -80°C and immediately placed on ice. After the cell pellet thawed for 5 minutes, the ligation product was added and mixed gently by tapping the bottom of the centrifuge tube, followed by incubation on ice for 25 minutes. Heat shock was performed in a 42°C water bath for 45 seconds, and the tube was immediately returned to ice and incubated for 2 minutes. 700 µL of antibiotic-free sterile LB medium was added to the centrifuge tube, mixed well, and the cells were recovered at 37°C, 200 rpm for 60 minutes. The cells were collected by centrifugation at 5000 rpm for 1 minute. Approximately 100 µL of the supernatant was retained to gently resuspend the cell pellet, which was then spread on LB medium supplemented with ampicillin. The plate was inverted and cultured overnight in a 37°C incubator. Single colonies were picked and verified by sequencing. Positive clones were cultured with shaking, and the chimeric recombinant plasmids were extracted using an Endo-Free Plasmid Maxiprep Kit (TIANGEN, DP120-01). The plasmid concentration was determined, and the plasmids were stored at -20°C for subsequent use.

### (5) Cell Culture and Transfection

HEK293T cells (purchased from ATCC) were seeded in DMEM medium (Gibco, 11965092) supplemented with 10% (v/v) fetal bovine serum (FBS) and 1% (v/v) Penicillin-Streptomycin (Gibco, 15140122), and cultured in a 37°C cell incubator with 5% CO₂. Cells for transfection were seeded in 24-well cell culture plates one day in advance; transfection was performed the next day when the cell density reached approximately 80%. The transfection dosage per well of the 24-well plate was 0.4 µg chimeric recombinant plasmid and 0.4 µg sgRNA plasmid, respectively. The PCSK9-sgRNA identical to that in Example 2 was used for activity testing.

The chimeric recombinant plasmid and sgRNA plasmid were mixed, diluted with 25 µL serum-reduced medium (Yuanpei Biotechnology, L530KJ), supplemented with 2 µL P3000 reagent, and mixed by pipetting to prepare Reagent A, which was incubated for 5 minutes. Meanwhile, 2 µL Lipofectamine 3000 transfection reagent (Thermo, 11668019) was diluted with 25 µL serum-reduced medium and mixed to prepare Reagent B, which was incubated for 5 minutes. Reagent A and Reagent B were mixed, homogenized by pipetting, and incubated for 20 minutes. After incubation, the mixed reagent was added dropwise to the cells in the 24-well plate to be transfected, and the plate was returned to the 37°C incubator for culture. The medium was replaced with DMEM medium containing 10% FBS 6 hours after transfection. Cells were harvested 48 hours after transfection for editing efficiency detection.

### (6) Editing Efficiency Detection

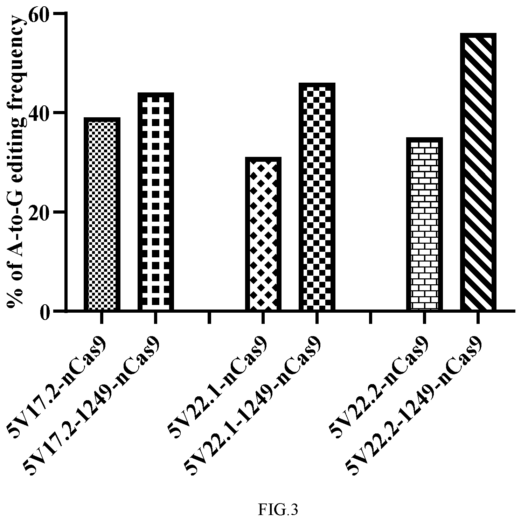

Genomic DNA was extracted from HEK293T cells using a Genomic DNA Extraction Kit (TIANGEN, DP304-03). The identification primer sequences and editing efficiency determination method were identical to those in Example 2. The editing efficiency of the chimeric base editors was also compared with that of the unmodified base editors. The base editing efficiencies of 5V17.2-1249-nCas9, 5V22.1-1249-nCas9 and 5V22.2-1249-nCas9 at the PCSK9 gene target site (Position A6) were 44%, 46% and 56%, respectively, representing a significant increase in base editing efficiency compared with the unmodified versions (39%, 31% and 35%, respectively) (FIG. 3).

### Example 4: Targeted Editing of α1-Antitrypsin Deficiency and Hemoglobinopathy Gene Targets Using Base Editors

HEK293T cells harboring the E342K mutation in the A1AT (α1-antitrypsin) gene were used for testing. The deaminase domain of plasmid pCMV-SpRY-ABE8e (Addgene, 185671) was replaced with the 5V17.9 variant as the test construct. The A1AT target sequence was designed as follows based on the target site: ATCGACAAGAAAGGGACTGA (SEQ ID NO: 42).

The A1AT-sgRNA plasmid was constructed in the same manner as Example 1, and co-transfected with the base editor 5V17.9-nCas9 into the aforementioned mutant HEK293T cells (experimental methods were identical to those in Example 3). PCR amplification was performed using primers (A1AT-F: SEQ ID NO: 43, A1AT-R: SEQ ID NO: 44), followed by editing efficiency detection. Detection and analysis showed that the base editor 5V17.9-nCas9 exhibited significant editing efficiency at the target site, with an editing efficiency of 51% for the A-to-G conversion at this position.

HEK293T cells harboring the E6V mutation in the β-globin gene were used for testing. The deaminase domain of plasmid pCMV-SpRY-ABE8e (Addgene, 185671) was replaced with the 5V17.9 variant as the test construct. The β-globin target sequence was designed as follows based on the target site: ACTTCTCCACAGGAGTCAGA (SEQ ID NO: 45).

The β-globin-sgRNA plasmid was constructed in the same manner as Example 1, and co-transfected with the base editor 5V17.9-nCas9 into the aforementioned mutant HEK293T cells (transfection methods were identical to those in Example 3). PCR amplification was performed using primers (β-globin-F: SEQ ID NO: 46, β-globin-R: SEQ ID NO: 47), followed by editing efficiency detection. Detection and analysis showed that the base editor 5V17.9-nCas9 exhibited significant editing efficiency at the target site, with an editing efficiency of 35% for the A-to-G conversion at this position.

Therefore, the base editors of the present disclosure can be used in the treatment of α1-antitrypsin deficiency and hemoglobinopathies.

### Example 5: Treatment of Base Mutation Diseases Using Base Editors

The base editors provided by the present disclosure (e.g., 5V17.2-nCas9) can be used to modify pathogenic DNA target sites. For example, a base editor can be used to site-directly mutate adenine (A) to guanine (G) in a nucleic acid (e.g., DNA). Such alterations change the amino acid sequence of a protein to disrupt or create a new initiation codon, or create a stop codon, to disrupt a splice donor, disrupt a splice acceptor, or edit a regulatory sequence, thereby achieving correction of a pathogenic gene for therapeutic purposes.

Said diseases are obtained from the NCBI ClinVar database available on the NCBI ClinVar website. For example, they may be selected from the disease targets for base editing shown in Table 34 of WO2022056254A2 published on March 17, 2022.

Partial sequences used in the present disclosure are shown in Table 8 below.

**Table 8. Partial sequences used in the present disclosure**

| SEQ ID NO. | Description |
|---|---|
| 1 | Amino acid sequence of deaminase 005V1 |
| | MSELNDAYWMKQALALAQKAREQGEVPVGAILVLDDEVIGQGWNRAITL HDPTAHAEIMALQQGGQIVQNYRLLNATLYVTFEPCVMCAGAMVHSRIKR LVYGVSNSKRGAAGSLLNVLNYPGMNHQIEITAGVMANECSEMLCQFYQ QPREVFNAEREARRLNQPDRAD |
| 2 | Nucleotide coding sequence of deaminase 005V1 |
| | ATGAGTGAGCTGAATGATGCTTACTGGATGAAACAGGCACTCGCTTTAG CTCAGAAGGCCCGGGAACAGGGAGAAGTTCCAGTGGGCGCTATTCTGG TGCTGGATGATGAAGTGATAGGACAGGGATGGAATAGAGCCATCACCCT GCACGACCCCACCGCCCACGCCGAGATCATGGCCCTGCAGCAGGGCGG CCAGATCGTGCAGAACTACCGGCTGCTGAACGCCACCCTGTACGTGACC TTCGAGCCCTGCGTGATGTGCGCCGGCGCCATGGTGCACAGCAGGATCA AGAGACTGGTCTACGGCGTGAGCAACTCAAAAAGAGGCGCCGCCGGC AGCCTGCTGAACGTGCTGAACTACCCCGGCATGAACCACCAGATCGAG ATCACCGCCGGCGTGATGGCCAACGAGTGCAGCGAGATGCTGTGCCAG TTCTATCAGCAGCCTAGGGAAGTGTTCAATGCTGAGCGTGAGGCTAGGC GGCTGAACCAACCTGATAGAGCTGAC |
| 3 | Protein sequence of 005V1-nCas9 |
| | MKRTADGSEFESPKKKRKVMSELNDAYWMKQALALAQKAREQGEVPVG AILVLDDEVIGQGWNRAITLHDPTAHAEIMALQQGGQIVQNYRLLNATLY VTFEPCVMCAGAMVHSRIKRLVYGVSNSKRGAAGSLLNVLNYPGMNHQI EITAGVMANECSEMLCQFYQQPREVFNAEREARRLNQPDRADSGGSSGGS SGSETPGTSESATPESSGGSSGGSDKKYSIGLAIGTNSVGWAVITDEYKVPS KKFKVLGNTDRHSIKKNLIGALLFDSGETAEATRLKRTARRRYTRRKNRIC YLQEIFSNEMAKVDDSFFHRLEESFLVEEDKKHERHPIFGNIVDEVAYHEK YPTIYHLRKKLVDSTDKADLRLIYLALAHMIKFRGHFLIEGDLNPDNSDVD KLFIQLVQTYNQLFEENPINASGVDAKAILSARLSKSRRLENLIAQLPGEKK NGLFGNLIALSLGLTPNFKSNFDLAEDAKLQLSKDTYDDDLDNLLAQIGDQ YADLFLAAKNLSDAILLSDILRVNTEITKAPLSASMIKRYDEHHQDLTLLKA LVRQQLPEKYKEIFFDQSKNGYAGYIDGGASQEEFYKFIKPILEKMDGTEEL LVKLNREDLLRKQRTFDNGSIPHQIHLGELHAILRRQEDFYPFLKDNREKIE KILTFRIPYYVGPLARGNSRFAWMTRKSEETITPWNFEEVVDKGASAQSFIE RMTNFDKNLPNEKVLPKHSLLYEYFTVYNELTKVKYVTEGMRKPAFLSGE QKKAIVDLLFKTNRKVTVKQLKEDYFKKIECFDSVEISGVEDRFNASLGTY HDLLKIIKDKDFLDNEENEDILEDIVLTLTLFEDREMIEERLKTYAHLFDDK VMKQLKRRRYTGWGRLSRKLINGIRDKQSGKTILDFLKSDGFANRNFMQL IHDDSLTFKEDIQKAQVSGQGDSLHEHIANLAGSPAIKKGILQTVKVVDELV KVMGRHKPENIVIEMARENQTTQKGQKNSRERMKRIEEGIKELGSQILKE HPVENTQLQNEKLYLYYLQNGRDMYVDQELDINRLSDYDVDHIVPQSFLK DDSIDNKVLTRSDKNRGKSDNVPSEEVVKKMKNYWRQLLNAKLITQRKF DNLTKAERGGLSELDKAGFIKRQLVETRQITKHVAQILDSRMNTKYDEND KLIREVKVITLKSKLVSDFRKDFQFYKVREINNYHHAHDAYLNAVVGTALI KKYPKLESEFVYGDYKVYDVRKMIAKSEQEIGKATAKYFFYSNIMNFFKT EITLANGEIRKRPLIETNGETGEIVWDKGRDFATVRKVLSMPQVNIVKKTE VQTGGFSKESILPKRNSDKLIARKKDWDPKKYGGFDSPTVAYSVLVVAKV EKGKSKKLKSVKELLGITIMERSSFEKNPIDFLEAKGYKEVKKDLIIKLPKY SLFELENGRKRMLASAGELQKGNELALPSKYVNFLYLASHYEKLKGSPED NEQKQLFVEQHKHYLDEIIEQISEFSKRVILADANLDKVLSAYNKHRDKPIR EQAENIIHLFTLTNLGAPAAFKYFDTTIDRKRYTSTKEVLDATLIHQSITGLY ETRIDLSQLGGDSGGSKRTADGSEFEPKKKRKV |
| | Nucleotide coding sequence of 005V1-nCas9 protein |
| 4 | atgaaacggacagccgacggaagcgagttcgagtcaccaaagaagaagcggaaagtcatgagtgagctgaatgat gcttactggatgaaacaggcactcgctttagctcagaaggcccgggaacagggagaagttccagtgggcgctattct ggtgctggatgatgaagtgataggacagggatggaatagagccatcaccctgcacgaccccaccgcccacgccga gatcatggccctgcagcagggcggccagatcgtgcagaactaccggctgctgaacgccaccctgtacgtgaccttc gagccctgcgtgatgtgcgccggcgccatggtgcacagcaggatcaagagactggtctacggcgtgagcaactcaa aaagaggcgccgccggcagcctgctgaacgtgctgaactaccccggcatgaaccaccagatcgagatcaccgccg gcgtgatggccaacgagtgcagcgagatgctgtgccagttctatcagcagcctagggaagtgttcaatgctgagcgt gaggctaggcggctgaaccaacctgatagagctgactccggaggatctagcggaggctcctctggctctgagacac ctggcacaagcgagagcgcaacacctgaaagcagcgggggcagcagcggggggtcagacaagaagtacagcat cggcctggccatcggcaccaactctgtgggctgggccgtgatcaccgacgagtacaaggtgcccagcaagaaattc aaggtgctgggcaacaccgaccggcacagcatcaagaagaacctgatcggagccctgctgttcgacagcggcgaa acagccgaggccacccggctgaagagaaccgccagaagaagatacaccagacggaagaaccggatctgctatct gcaagagatcttcagcaacgagatggccaaggtggacgacagcttcttccacagactggaagagtccttcctggtgg aagaggataagaagcacgagcggcaccccatcttcggcaacatcgtggacgaggtggcctaccacgagaagtacc ccaccatctaccacctgagaaagaaactggtggacagcaccgacaaggccgacctgcggctgatctatctggccct ggcccacatgatcaagttccggggccacttcctgatcgagggcgacctgaaccccgacaacagcgacgtggacaa gctgttcatccagctggtgcagacctacaaccagctgttcgaggaaaaccccatcaacgccagcggcgtggacgcc aaggccatcctgtctgccagactgagcaagagcagacggctggaaaatctgatcgcccagctgcccggcgagaag aagaatggcctgttcggaaacctgattgccctgagcctgggcctgacccccaacttcaagagcaacttcgacctggcc gaggatgccaaactgcagctgagcaaggacacctacgacgacgacctggacaacctgctggcccagatcggcgac cagtacgccgacctgtttctggccgccaagaacctgtccgacgccatcctgctgagcgacatcctgagagtgaacac cgagatcaccaaggcccccctgagcgcctctatgatcaagagatacgacgagcaccaccaggacctgaccctgctg aaagctctcgtgcggcagcagctgcctgagaagtacaaagagattttcttcgaccagagcaagaacggctacgccg gctacattgacggcggagccagccaggaagagttctacaagttcatcaagcccatcctggaaaagatggacggcac cgaggaactgctcgtgaagctgaacagagaggacctgctgcggaagcagcggaccttcgacaacggcagcatccc ccaccagatccacctgggagagctgcacgccattctgcggcggcaggaagatttttacccattcctgaaggacaacc gggaaaagatcgagaagatcctgaccttccgcatcccctactacgtgggccctctggccaggggaaacagcagattc gcctggatgaccagaaagagcgaggaaaccatcaccccctggaacttcgaggaagtggtggacaagggcgcttcc gcccagagcttcatcgagcggatgaccaacttcgataagaacctgcccaacgagaaggtgctgcccaagcacagcc tgctgtacgagtacttcaccgtgtataacgagctgaccaaagtgaaatacgtgaccgagggaatgagaaagcccgcc ttcctgagcggcgagcagaaaaaggccatcgtggacctgctgttcaagaccaaccggaaagtgaccgtgaagcagc tgaaagaggactacttcaagaaaatcgagtgcttcgactccgtggaaatctccggcgtggaagatcggttcaacgcct ccctgggcacataccacgatctgctgaaaattatcaaggacaaggacttcctggacaatgaggaaaacgaggacatt ctggaagatatcgtgctgaccctgacactgtttgaggacagagagatgatcgaggaacggctgaaaacctatgccca cctgttcgacgacaaagtgatgaagcagctgaagcggcggagatacaccggctggggcaggctgagccggaagct gatcaacggcatccgggacaagcagtccggcaagacaatcctggatttcctgaagtccgacggcttcgccaacaga aacttcatgcagctgatccacgacgacagcctgacctttaaagaggacatccagaaagcccaggtgtccggccagg gcgatagcctgcacgagcacattgccaatctggccggcagccccgccattaagaagggcatcctgcagacagtgaa ggtggtggacgagctcgtgaaagtgatgggccggcacaagcccgagaacatcgtgatcgaaatggccagagagaa ccagaccacccagaagggacagaagaacagccgcgagagaatgaagcggatcgaagagggcatcaaagagctg ggcagccagatcctgaaagaacaccccgtggaaaacacccagctgcagaacgagaagctgtacctgtactacctgc agaatgggcgggatatgtacgtggaccaggaactggacatcaaccggctgtccgactacgatgtggaccatatcgtg cctcagagctttctgaaggacgactccatcgacaacaaggtgctgaccagaagcgacaagaaccggggcaagagc gacaacgtgccctccgaagaggtcgtgaagaagatgaagaactactggcggcagctgctgaacgccaagctgatta cccagagaaagttcgacaatctgaccaaggccgagagaggcggcctgagcgaactggataaggccggcttcatca agagacagctggtggaaacccggcagatcacaaagcacgtggcacagatcctggactcccggatgaacactaagt acgacgagaatgacaagctgatccgggaagtgaaagtgatcaccctgaagtccaagctggtgtccgatttccggaag gatttccagttttacaaagtgcgcgagatcaacaactaccaccacgcccacgacgcctacctgaacgccgtcgtggg aaccgccctgatcaaaaagtaccctaagctggaaagcgagttcgtgtacggcgactacaaggtgtacgacgtgcgg aagatgatcgccaagagcgagcaggaaatcggcaaggctaccgccaagtacttcttctacagcaacatcatgaacttt ttcaagaccgagattaccctggccaacggcgagatccggaagcggcctctgatcgagacaaacggcgaaaccggg |
| | gagatcgtgtgggataagggccgggattttgccaccgtgcggaaagtgctgagcatgccccaagtgaatatcgtgaa aaagaccgaggtgcagacaggcggcttcagcaaagagtctatcctgcccaagaggaacagcgataagctgatcgc cagaaagaaggactgggaccctaagaagtacggcggcttcgacagccccaccgtggcctattctgtgctggtggtg gccaaagtggaaaagggcaagtccaagaaactgaagagtgtgaaagagctgctggggatcaccatcatggaaaga agcagcttcgagaagaatcccatcgactttctggaagccaagggctacaaagaagtgaaaaaggacctgatcatcaa gctgcctaagtactccctgttcgagctggaaaacggccggaagagaatgctggcctctgccggcgaactgcagaag ggaaacgaactggccctgccctccaaatatgtgaacttcctgtacctggccagccactatgagaagctgaagggctcc cccgaggataatgagcagaaacagctgtttgtggaacagcacaagcactacctggacgagatcatcgagcagatca gcgagttctccaagagagtgatcctggccgacgctaatctggacaaagtgctgtccgcctacaacaagcaccgggat aagcccatcagagagcaggccgagaatatcatccacctgtttaccctgaccaatctgggagcccctgccgccttcaa gtactttgacaccaccatcgaccggaagaggtacaccagcaccaaagaggtgctggacgccaccctgatccaccag agcatcaccggcctgtacgagacacggatcgacctgtctcagctgggaggtgactctggcggctcaaaaagaaccg ccgacggcagcgaattcgagcccaagaagaagaggaaagtc |
| 5 | PCSK9 target sequence |
| | Cccgcaccttggcgcagcgg |
| 6 | Forward primer for PCSK9-sgRNA (PCSK9-sgRNA-F) |
| | CACCcccgcaccttggcgcagcgg |
| 7 | Reverse primer for PCSK9-sgRNA (PCSK9-sgRNA-R) |
| | AAACccgctgcgccaaggtgcggg |
| 8 | Forward primer for PCSK9 (PCSK9-F) |
| | ggtgctagccttgcgttccg |
| 9 | Reverse primer for PCSK9 (PCSK9-R) |
| | gtccccaagatcgtgccaa |
| 10 | Amino acid sequence of deaminase 5V3354 |
| | MSELNDAYWMKQALALAQKAREQGEVPVGAILVLDDEVIGQGWNRCYG HHDPTAHAEIMALQQGGQIVQNYRLLNATLYVTFEPCVMCAGAMVHSRI KRLVYGMSNSKRGAAGSLLNVLNYPGMNHQIEITAGVMANECSEMLCQF YQQPREVFNAEREARRLNQPDRAD |
| 11 | Nucleotide coding sequence of deaminase 5V3354 |
| | ATGAGTGAGCTGAATGATGCTTACTGGATGAAACAGGCACTCGCTTTAG CTCAGAAGGCCcggGAACAGGGAGAAGTTCCAGTGGGCGCTATTCTGGT GctgGATGATGAAGTGATAGGACAGGGATGGAATAGATGCTACGGCCATC ACGACCCCACCGCCCACGCCGAGATCATGGCCCTGCAGCAGGGCGGCC AGATCGTGCAGAACTACCGGCTGCTGAACGCCACCCTGTACGTGACCttc GAGCCCTGCGTGATGTGCGCCGGCGCCATGGTGCACAGCAGGATCAAG AGACTGGTCTACGGCATGAGCAACtcaAAAagaGGCGCCGCCGGCAGCCT GCTGaacGTGCTGaactacCCCGGCATGAACCACCAGATCGAGATCACCGCC GGCGTGATGGCCAACGAGTGCAGCGAGATGCTGtgcCAGTTCtatCAGCAG cctAGGGAAgtgttcaatGCTGAGCGTGAGGCTAGGCGGCTGAACCAACCTGA TAGAGCTGAC |
| 12 | Forward primer for nCas9 (nCas9-F) |
| | gaagcggaaagtcgacaagaagtacagcatcggcc |
| 13 | Reverse primer for nCas9 (nCas9-R) |
| | ctgtacttcttgtcgactttccgcttcttctttggtgac |
| 14 | Forward primer for ADA (ADA-F) |
| | TCTGGAGGATCTAGCGGCGGATCCTCTGGAAGCGAGACACCAGGCACA AGCGAGTCCGCCACACCAGAGAGCTCCGGCGGCTCCTCCGGAGGATCC AGTGAGCTGAATGATGC |
| 15 | Reverse primer for ADA (ADA-R) |
| | tgaccccccgctgctgcccccgctgctttcaggtgttgcg |
| 16 | Forward primer for nCas9-1249 (nCas9-1249-F) |
| | ggggcagcagcggggggtcacccgaggataatgagcagaaacagctgt |
| 17 | Reverse primer for nCas9-1249 (nCas9-1249-R) |
| | CCGCCGCTAGATCCTCCAGAggagcccttcagcttctcatagtggct |
| 18 | Amino acid sequence of 5V17.2-1249-nCas9 |
| | MKRTADGSEFESPKKKRKVMDKKYSIGLAIGTNSVGWAVITDEYKVPSKK FKVLGNTDRHSIKKNLIGALLFDSGETAEATRLKRTARRRYTRRKNRICYL QEIFSNEMAKVDDSFFHRLEESFLVEEDKKHERHPIFGNIVDEVAYHEKYPT IYHLRKKLVDSTDKADLRLIYLALAHMIKFRGHFLIEGDLNPDNSDVDKLF IQLVQTYNQLFEENPINASGVDAKAILSARLSKSRRLENLIAQLPGEKKNGL FGNLIALSLGLTPNFKSNFDLAEDAKLQLSKDTYDDDLDNLLAQIGDQYAD LFLAAKNLSDAILLSDILRVNTEITKAPLSASMIKRYDEHHQDLTLLKALVR QQLPEKYKEIFFDQSKNGYAGYIDGGASQEEFYKFIKPILEKMDGTEELLV KLNREDLLRKQRTFDNGSIPHQIHLGELHAILRRQEDFYPFLKDNREKIEKI LTFRIPYYVGPLARGNSRFAWMTRKSEETITPWNFEEVVDKGASAQSFIER MTNFDKNLPNEKVLPKHSLLYEYFTVYNELTKVKYVTEGMRKPAFLSGEQ KKAIVDLLFKTNRKVTVKQLKEDYFKKIECFDSVEISGVEDRFNASLGTYH DLLKIIKDKDFLDNEENEDILEDIVLTLTLFEDREMIEERLKTYAHLFDDKV MKQLKRRRYTGWGRLSRKLINGIRDKQSGKTILDFLKSDGFANRNFMQLI HDDSLTFKEDIQKAQVSGQGDSLHEHIANLAGSPAIKKGILQTVKVVDELV KVMGRHKPENIVIEMARENQTTQKGQKNSRERMKRIEEGIKELGSQILKE HPVENTQLQNEKLYLYYLQNGRDMYVDQELDINRLSDYDVDHIVPQSFLK DDSIDNKVLTRSDKNRGKSDNVPSEEVVKKMKNYWRQLLNAKLITQRKF DNLTKAERGGLSELDKAGFIKRQLVETRQITKHVAQILDSRMNTKYDEND KLIREVKVITLKSKLVSDFRKDFQFYKVREINNYHHAHDAYLNAVVGTALI KKYPKLESEFVYGDYKVYDVRKMIAKSEQEIGKATAKYFFYSNIMNFFKT EITLANGEIRKRPLIETNGETGEIVWDKGRDFATVRKVLSMPQVNIVKKTE VQTGGFSKESILPKRNSDKLIARKKDWDPKKYGGFDSPTVAYSVLVVAKV EKGKSKKLKSVKELLGITIMERSSFEKNPIDFLEAKGYKEVKKDLIIKLPKY SLFELENGRKRMLASAGELQKGNELALPSKYVNFLYLASHYEKLKGSSGG SSGGSSGSETPGTSESATPESSGGSSGGSSELNDAYWMKQALALAQKAREQ GEVPVGAILVLDDEVIGQGWNRCYGHHDPTAHAEIMALQQGGQIVQNYR LLNATLYVTFEPCVMCAGAMVHSRIKRLVYGMSNSKRGAAGSLLNVLNY PGMNHQIEITAGVMANECSEMLCQFYRQLRLAPGAEREARRLNQPDRADS GGSSGGSSGSETPGTSESATPESSGGSSGGSPEDNEQKQLFVEQHKHYLDEI IEQISEFSKRVILADANLDKVLSAYNKHRDKPIREQAENIIHLFTLTNLGAPA AFKYFDTTIDRKRYTSTKEVLDATLIHQSITGLYETRIDLSQLGGD* |
| | Nucleotide coding sequence of 5V17.2-1249-nCas9 |
| 19 | atgaaacggacagccgacggaagcgagttcgagtcaccaaagaagaagcggaaagtcATGgacaagaagtaca gcatcggcctggccatcggcaccaactctgtgggctgggccgtgatcaccgacgagtacaaggtgcccagcaaga aattcaaggtgctgggcaacaccgaccggcacagcatcaagaagaacctgatcggagccctgctgttcgacagcgg cgaaacagccgaggccacccggctgaagagaaccgccagaagaagatacaccagacggaagaaccggatctgct atctgcaagagatcttcagcaacgagatggccaaggtggacgacagcttcttccacagactggaagagtccttcctgg tggaagaggataagaagcacgagcggcaccccatcttcggcaacatcgtggacgaggtggcctaccacgagaagt accccaccatctaccacctgagaaagaaactggtggacagcaccgacaaggccgacctgcggctgatctatctggc cctggcccacatgatcaagttccggggccacttcctgatcgagggcgacctgaaccccgacaacagcgacgtggac aagctgttcatccagctggtgcagacctacaaccagctgttcgaggaaaaccccatcaacgccagcggcgtggacg ccaaggccatcctgtctgccagactgagcaagagcagacggctggaaaatctgatcgcccagctgcccggcgaga agaagaatggcctgttcggaaacctgattgccctgagcctgggcctgacccccaacttcaagagcaacttcgacctg gccgaggatgccaaactgcagctgagcaaggacacctacgacgacgacctggacaacctgctggcccagatcggc gaccagtacgccgacctgtttctggccgccaagaacctgtccgacgccatcctgctgagcgacatcctgagagtgaa caccgagatcaccaaggcccccctgagcgcctctatgatcaagagatacgacgagcaccaccaggacctgaccctg ctgaaagctctcgtgcggcagcagctgcctgagaagtacaaagagattttcttcgaccagagcaagaacggctacgc cggctacattgacggcggagccagccaggaagagttctacaagttcatcaagcccatcctggaaaagatggacggc accgaggaactgctcgtgaagctgaacagagaggacctgctgcggaagcagcggaccttcgacaacggcagcatc ccccaccagatccacctgggagagctgcacgccattctgcggcggcaggaagatttttacccattcctgaaggacaa ccgggaaaagatcgagaagatcctgaccttccgcatcccctactacgtgggccctctggccaggggaaacagcaga ttcgcctggatgaccagaaagagcgaggaaaccatcaccccctggaacttcgaggaagtggtggacaagggcgctt ccgcccagagcttcatcgagcggatgaccaacttcgataagaacctgcccaacgagaaggtgctgcccaagcacag cctgctgtacgagtacttcaccgtgtataacgagctgaccaaagtgaaatacgtgaccgagggaatgagaaagcccg ccttcctgagcggcgagcagaaaaaggccatcgtggacctgctgttcaagaccaaccggaaagtgaccgtgaagca gctgaaagaggactacttcaagaaaatcgagtgcttcgactccgtggaaatctccggcgtggaagatcggttcaacgc ctccctgggcacataccacgatctgctgaaaattatcaaggacaaggacttcctggacaatgaggaaaacgaggaca ttctggaagatatcgtgctgaccctgacactgtttgaggacagagagatgatcgaggaacggctgaaaacctatgccc acctgttcgacgacaaagtgatgaagcagctgaagcggcggagatacaccggctggggcaggctgagccggaag ctgatcaacggcatccgggacaagcagtccggcaagacaatcctggatttcctgaagtccgacggcttcgccaacag aaacttcatgcagctgatccacgacgacagcctgacctttaaagaggacatccagaaagcccaggtgtccggccag ggcgatagcctgcacgagcacattgccaatctggccggcagccccgccattaagaagggcatcctgcagacagtga aggtggtggacgagctcgtgaaagtgatgggccggcacaagcccgagaacatcgtgatcgaaatggccagagaga accagaccacccagaagggacagaagaacagccgcgagagaatgaagcggatcgaagagggcatcaaagagct gggcagccagatcctgaaagaacaccccgtggaaaacacccagctgcagaacgagaagctgtacctgtactacctg cagaatgggcgggatatgtacgtggaccaggaactggacatcaaccggctgtccgactacgatgtggaccatatcgt gcctcagagctttctgaaggacgactccatcgacaacaaggtgctgaccagaagcgacaagaaccggggcaagag cgacaacgtgccctccgaagaggtcgtgaagaagatgaagaactactggcggcagctgctgaacgccaagctgatt acccagagaaagttcgacaatctgaccaaggccgagagaggcggcctgagcgaactggataaggccggcttcatc aagagacagctggtggaaacccggcagatcacaaagcacgtggcacagatcctggactcccggatgaacactaag tacgacgagaatgacaagctgatccgggaagtgaaagtgatcaccctgaagtccaagctggtgtccgatttccggaa ggatttccagttttacaaagtgcgcgagatcaacaactaccaccacgcccacgacgcctacctgaacgccgtcgtgg gaaccgccctgatcaaaaagtaccctaagctggaaagcgagttcgtgtacggcgactacaaggtgtacgacgtgcg gaagatgatcgccaagagcgagcaggaaatcggcaaggctaccgccaagtacttcttctacagcaacatcatgaact ttttcaagaccgagattaccctggccaacggcgagatccggaagcggcctctgatcgagacaaacggcgaaaccgg ggagatcgtgtgggataagggccgggattttgccaccgtgcggaaagtgctgagcatgccccaagtgaatatcgtga aaaagaccgaggtgcagacaggcggcttcagcaaagagtctatcctgcccaagaggaacagcgataagctgatcg ccagaaagaaggactgggaccctaagaagtacggcggcttcgacagccccaccgtggcctattctgtgctggtggt ggccaaagtggaaaagggcaagtccaagaaactgaagagtgtgaaagagctgctggggatcaccatcatggaaag aagcagcttcgagaagaatcccatcgactttctggaagccaagggctacaaagaagtgaaaaaggacctgatcatca agctgcctaagtactccctgttcgagctggaaaacggccggaagagaatgctggcctctgccggcgaactgcagaa gggaaacgaactggccctgccctccaaatatgtgaacttcctgtacctggccagccactatgagaagctgaagggct ccTCTGGAGGATCTAGCGGCGGATCCTCTGGAAGCGAGACACCAGGCAC |
| | AAGCGAGTCCGCCACACCAGAGAGCTCCGGCGGCTCCTCCGGAGGATC CAGTGAGCTGAATGATGCTTACTGGATGAAACAGGCACTCGCTTTAGCT CAGAAGGCCcggGAACAGGGAGAAGTTCCAGTGGGCGCTATTCTGGTGct gGATGATGAAGTGATAGGACAGGGATGGAATAGATGCTACGGCCATCAC GACCCCACCGCCCACGCCGAGATCATGGCCCTGCAGCAGGGCGGCCAG ATCGTGCAGAACTACCGGCTGCTGAACGCCACCCTGTACGTGACCttcGA GCCCTGCGTGATGTGCGCCGGCGCCATGGTGCACAGCAGGATCAAGAG ACTGGTCTACGGCATGAGCAACtcaAAAagaGGCGCCGCCGGCAGCCTGC TGaacGTGCTGaactacCCCGGCATGAACCACCAGATCGAGATCACCGCCGG CGTGATGGCCAACGAGTGCAGCGAGATGCTGtgcCAGTTCtatCGACAGTT GAGGCTCGCCCCCGGGGCTGAGCGTGAGGCTAGGCGGCTGAACCAACC TGATAGAGCTGACtccggaggatctagcggaggctcctctggctctgagacacctggcacaagcgagag cgcaacacctgaaagcagcgggggcagcagcggggggtcacccgaggataatgagcagaaacagctgtttgtgg aacagcacaagcactacctggacgagatcatcgagcagatcagcgagttctccaagagagtgatcctggccgacgc taatctggacaaagtgctgtccgcctacaacaagcaccgggataagcccatcagagagcaggccgagaatatcatcc acctgtttaccctgaccaatctgggagcccctgccgccttcaagtactttgacaccaccatcgaccggaagaggtaca ccagcaccaaagaggtgctggacgccaccctgatccaccagagcatcaccggcctgtacgagacacggatcgacc tgtctcagctgggaggtgac |
| | Amino acid sequence of 5V22.1-1249-nCas9 |
| 20 | MKRTADGSEFESPKKKRKVMDKKYSIGLAIGTNSVGWAVITDEYKVPSKK FKVLGNTDRHSIKKNLIGALLFDSGETAEATRLKRTARRRYTRRKNRICYL QEIFSNEMAKVDDSFFHRLEESFLVEEDKKHERHPIFGNIVDEVAYHEKYPT IYHLRKKLVDSTDKADLRLIYLALAHMIKFRGHFLIEGDLNPDNSDVDKLF IQLVQTYNQLFEENPINASGVDAKAILSARLSKSRRLENLIAQLPGEKKNGL FGNLIALSLGLTPNFKSNFDLAEDAKLQLSKDTYDDDLDNLLAQIGDQYAD LFLAAKNLSDAILLSDILRVNTEITKAPLSASMIKRYDEHHQDLTLLKALVR QQLPEKYKEIFFDQSKNGYAGYIDGGASQEEFYKFIKPILEKMDGTEELLV KLNREDLLRKQRTFDNGSIPHQIHLGELHAILRRQEDFYPFLKDNREKIEKI LTFRIPYYVGPLARGNSRFAWMTRKSEETITPWNFEEVVDKGASAQSFIER MTNFDKNLPNEKVLPKHSLLYEYFTVYNELTKVKYVTEGMRKPAFLSGEQ KKAIVDLLFKTNRKVTVKQLKEDYFKKIECFDSVEISGVEDRFNASLGTYH DLLKIIKDKDFLDNEENEDILEDIVLTLTLFEDREMIEERLKTYAHLFDDKV MKQLKRRRYTGWGRLSRKLINGIRDKQSGKTILDFLKSDGFANRNFMQLI HDDSLTFKEDIQKAQVSGQGDSLHEHIANLAGSPAIKKGILQTVKVVDELV KVMGRHKPENIVIEMARENQTTQKGQKNSRERMKRIEEGIKELGSQILKE HPVENTQLQNEKLYLYYLQNGRDMYVDQELDINRLSDYDVDHIVPQSFLK DDSIDNKVLTRSDKNRGKSDNVPSEEVVKKMKNYWRQLLNAKLITQRKF DNLTKAERGGLSELDKAGFIKRQLVETRQITKHVAQILDSRMNTKYDEND KLIREVKVITLKSKLVSDFRKDFQFYKVREINNYHHAHDAYLNAVVGTALI KKYPKLESEFVYGDYKVYDVRKMIAKSEQEIGKATAKYFFYSNIMNFFKT EITLANGEIRKRPLIETNGETGEIVWDKGRDFATVRKVLSMPQVNIVKKTE VQTGGFSKESILPKRNSDKLIARKKDWDPKKYGGFDSPTVAYSVLVVAKV EKGKSKKLKSVKELLGITIMERSSFEKNPIDFLEAKGYKEVKKDLIIKLPKY SLFELENGRKRMLASAGELQKGNELALPSKYVNFLYLASHYEKLKGSSGG SSGGSSGSETPGTSESATPESSGGSSGGSSELNDAYWMKQALALAQKAREQ GEVPVGAILVLDDEVIGQGWNRCYAHHDPTAHAEIMALQQGGQIVQNYR LLNATLYVTFEPCVMCAGAMVHSRIKRLVYGMSNSKRGAAGSLLNVLNY PGMNHQIEITAGVMANECSEMLCQFYRQLRLAPGAEREARRLNQPDRADS GGSSGGSSGSETPGTSESATPESSGGSSGGSPEDNEQKQLFVEQHKHYLDEI IEQISEFSKRVILADANLDKVLSAYNKHRDKPIREQAENIIHLFTLTNLGAPA AFKYFDTTIDRKRYTSTKEVLDATLIHQSITGLYETRIDLSQLGGD |
| | Nucleotide coding sequence of 5V22.1-1249-nCas9 |
| 21 | atgaaacggacagccgacggaagcgagttcgagtcaccaaagaagaagcggaaagtcATGgacaagaagtaca gcatcggcctggccatcggcaccaactctgtgggctgggccgtgatcaccgacgagtacaaggtgcccagcaaga aattcaaggtgctgggcaacaccgaccggcacagcatcaagaagaacctgatcggagccctgctgttcgacagcgg cgaaacagccgaggccacccggctgaagagaaccgccagaagaagatacaccagacggaagaaccggatctgct atctgcaagagatcttcagcaacgagatggccaaggtggacgacagcttcttccacagactggaagagtccttcctgg tggaagaggataagaagcacgagcggcaccccatcttcggcaacatcgtggacgaggtggcctaccacgagaagt accccaccatctaccacctgagaaagaaactggtggacagcaccgacaaggccgacctgcggctgatctatctggc cctggcccacatgatcaagttccggggccacttcctgatcgagggcgacctgaaccccgacaacagcgacgtggac aagctgttcatccagctggtgcagacctacaaccagctgttcgaggaaaaccccatcaacgccagcggcgtggacg ccaaggccatcctgtctgccagactgagcaagagcagacggctggaaaatctgatcgcccagctgcccggcgaga agaagaatggcctgttcggaaacctgattgccctgagcctgggcctgacccccaacttcaagagcaacttcgacctg gccgaggatgccaaactgcagctgagcaaggacacctacgacgacgacctggacaacctgctggcccagatcggc gaccagtacgccgacctgtttctggccgccaagaacctgtccgacgccatcctgctgagcgacatcctgagagtgaa caccgagatcaccaaggcccccctgagcgcctctatgatcaagagatacgacgagcaccaccaggacctgaccctg ctgaaagctctcgtgcggcagcagctgcctgagaagtacaaagagattttcttcgaccagagcaagaacggctacgc cggctacattgacggcggagccagccaggaagagttctacaagttcatcaagcccatcctggaaaagatggacggc accgaggaactgctcgtgaagctgaacagagaggacctgctgcggaagcagcggaccttcgacaacggcagcatc ccccaccagatccacctgggagagctgcacgccattctgcggcggcaggaagatttttacccattcctgaaggacaa ccgggaaaagatcgagaagatcctgaccttccgcatcccctactacgtgggccctctggccaggggaaacagcaga ttcgcctggatgaccagaaagagcgaggaaaccatcaccccctggaacttcgaggaagtggtggacaagggcgctt ccgcccagagcttcatcgagcggatgaccaacttcgataagaacctgcccaacgagaaggtgctgcccaagcacag cctgctgtacgagtacttcaccgtgtataacgagctgaccaaagtgaaatacgtgaccgagggaatgagaaagcccg ccttcctgagcggcgagcagaaaaaggccatcgtggacctgctgttcaagaccaaccggaaagtgaccgtgaagca gctgaaagaggactacttcaagaaaatcgagtgcttcgactccgtggaaatctccggcgtggaagatcggttcaacgc ctccctgggcacataccacgatctgctgaaaattatcaaggacaaggacttcctggacaatgaggaaaacgaggaca ttctggaagatatcgtgctgaccctgacactgtttgaggacagagagatgatcgaggaacggctgaaaacctatgccc acctgttcgacgacaaagtgatgaagcagctgaagcggcggagatacaccggctggggcaggctgagccggaag ctgatcaacggcatccgggacaagcagtccggcaagacaatcctggatttcctgaagtccgacggcttcgccaacag aaacttcatgcagctgatccacgacgacagcctgacctttaaagaggacatccagaaagcccaggtgtccggccag ggcgatagcctgcacgagcacattgccaatctggccggcagccccgccattaagaagggcatcctgcagacagtga aggtggtggacgagctcgtgaaagtgatgggccggcacaagcccgagaacatcgtgatcgaaatggccagagaga accagaccacccagaagggacagaagaacagccgcgagagaatgaagcggatcgaagagggcatcaaagagct gggcagccagatcctgaaagaacaccccgtggaaaacacccagctgcagaacgagaagctgtacctgtactacctg cagaatgggcgggatatgtacgtggaccaggaactggacatcaaccggctgtccgactacgatgtggaccatatcgt gcctcagagctttctgaaggacgactccatcgacaacaaggtgctgaccagaagcgacaagaaccggggcaagag cgacaacgtgccctccgaagaggtcgtgaagaagatgaagaactactggcggcagctgctgaacgccaagctgatt acccagagaaagttcgacaatctgaccaaggccgagagaggcggcctgagcgaactggataaggccggcttcatc aagagacagctggtggaaacccggcagatcacaaagcacgtggcacagatcctggactcccggatgaacactaag tacgacgagaatgacaagctgatccgggaagtgaaagtgatcaccctgaagtccaagctggtgtccgatttccggaa ggatttccagttttacaaagtgcgcgagatcaacaactaccaccacgcccacgacgcctacctgaacgccgtcgtgg gaaccgccctgatcaaaaagtaccctaagctggaaagcgagttcgtgtacggcgactacaaggtgtacgacgtgcg gaagatgatcgccaagagcgagcaggaaatcggcaaggctaccgccaagtacttcttctacagcaacatcatgaact ttttcaagaccgagattaccctggccaacggcgagatccggaagcggcctctgatcgagacaaacggcgaaaccgg ggagatcgtgtgggataagggccgggattttgccaccgtgcggaaagtgctgagcatgccccaagtgaatatcgtga aaaagaccgaggtgcagacaggcggcttcagcaaagagtctatcctgcccaagaggaacagcgataagctgatcg ccagaaagaaggactgggaccctaagaagtacggcggcttcgacagccccaccgtggcctattctgtgctggtggt ggccaaagtggaaaagggcaagtccaagaaactgaagagtgtgaaagagctgctggggatcaccatcatggaaag aagcagcttcgagaagaatcccatcgactttctggaagccaagggctacaaagaagtgaaaaaggacctgatcatca agctgcctaagtactccctgttcgagctggaaaacggccggaagagaatgctggcctctgccggcgaactgcagaa gggaaacgaactggccctgccctccaaatatgtgaacttcctgtacctggccagccactatgagaagctgaagggct ccTCTGGAGGATCTAGCGGCGGATCCTCTGGAAGCGAGACACCAGGCAC |
| | AAGCGAGTCCGCCACACCAGAGAGCTCCGGCGGCTCCTCCGGAGGATC CAGTGAGCTGAATGATGCTTACTGGATGAAACAGGCACTCGCTTTAGCT CAGAAGGCCcggGAACAGGGAGAAGTTCCAGTGGGCGCTATTCTGGTGct gGATGATGAAGTGATAGGACAGGGATGGAATAGATGCTACGCCCATCAC GACCCCACCGCCCACGCCGAGATCATGGCCCTGCAGCAGGGCGGCCAG ATCGTGCAGAACTACCGGCTGCTGAACGCCACCCTGTACGTGACCttcGA GCCCTGCGTGATGTGCGCCGGCGCCATGGTGCACAGCAGGATCAAGAG ACTGGTCTACGGCATGAGCAACtcaAAAagaGGCGCCGCCGGCAGCCTGC TGaacGTGCTGaactacCCCGGCATGAACCACCAGATCGAGATCACCGCCGG CGTGATGGCCAACGAGTGCAGCGAGATGCTGtgcCAGTTCtatCGACAGTT GAGGCTCGCCCCCGGGGCTGAGCGTGAGGCTAGGCGGCTGAACCAACC TGATAGAGCTGACtccggaggatctagcggaggctcctctggctctgagacacctggcacaagcgagag cgcaacacctgaaagcagcgggggcagcagcggggggtcacccgaggataatgagcagaaacagctgtttgtgg aacagcacaagcactacctggacgagatcatcgagcagatcagcgagttctccaagagagtgatcctggccgacgc taatctggacaaagtgctgtccgcctacaacaagcaccgggataagcccatcagagagcaggccgagaatatcatcc acctgtttaccctgaccaatctgggagcccctgccgccttcaagtactttgacaccaccatcgaccggaagaggtaca ccagcaccaaagaggtgctggacgccaccctgatccaccagagcatcaccggcctgtacgagacacggatcgacc tgtctcagctgggaggtgac |
| | Amino acid sequence of 5V22.2-1249-nCas9 |
| 22 | MKRTADGSEFESPKKKRKVMDKKYSIGLAIGTNSVGWAVITDEYKVPSKK FKVLGNTDRHSIKKNLIGALLFDSGETAEATRLKRTARRRYTRRKNRICYL QEIFSNEMAKVDDSFFHRLEESFLVEEDKKHERHPIFGNIVDEVAYHEKYPT IYHLRKKLVDSTDKADLRLIYLALAHMIKFRGHFLIEGDLNPDNSDVDKLF IQLVQTYNQLFEENPINASGVDAKAILSARLSKSRRLENLIAQLPGEKKNGL FGNLIALSLGLTPNFKSNFDLAEDAKLQLSKDTYDDDLDNLLAQIGDQYAD LFLAAKNLSDAILLSDILRVNTEITKAPLSASMIKRYDEHHQDLTLLKALVR QQLPEKYKEIFFDQSKNGYAGYIDGGASQEEFYKFIKPILEKMDGTEELLV KLNREDLLRKQRTFDNGSIPHQIHLGELHAILRRQEDFYPFLKDNREKIEKI LTFRIPYYVGPLARGNSRFAWMTRKSEETITPWNFEEVVDKGASAQSFIER MTNFDKNLPNEKVLPKHSLLYEYFTVYNELTKVKYVTEGMRKPAFLSGEQ KKAIVDLLFKTNRKVTVKQLKEDYFKKIECFDSVEISGVEDRFNASLGTYH DLLKIIKDKDFLDNEENEDILEDIVLTLTLFEDREMIEERLKTYAHLFDDKV MKQLKRRRYTGWGRLSRKLINGIRDKQSGKTILDFLKSDGFANRNFMQLI HDDSLTFKEDIQKAQVSGQGDSLHEHIANLAGSPAIKKGILQTVKVVDELV KVMGRHKPENIVIEMARENQTTQKGQKNSRERMKRIEEGIKELGSQILKE HPVENTQLQNEKLYLYYLQNGRDMYVDQELDINRLSDYDVDHIVPQSFLK DDSIDNKVLTRSDKNRGKSDNVPSEEVVKKMKNYWRQLLNAKLITQRKF DNLTKAERGGLSELDKAGFIKRQLVETRQITKHVAQILDSRMNTKYDEND KLIREVKVITLKSKLVSDFRKDFQFYKVREINNYHHAHDAYLNAVVGTALI KKYPKLESEFVYGDYKVYDVRKMIAKSEQEIGKATAKYFFYSNIMNFFKT EITLANGEIRKRPLIETNGETGEIVWDKGRDFATVRKVLSMPQVNIVKKTE VQTGGFSKESILPKRNSDKLIARKKDWDPKKYGGFDSPTVAYSVLVVAKV EKGKSKKLKSVKELLGITIMERSSFEKNPIDFLEAKGYKEVKKDLIIKLPKY SLFELENGRKRMLASAGELQKGNELALPSKYVNFLYLASHYEKLKGSSGG SSGGSSGSETPGTSESATPESSGGSSGGSSELNDAYWMKQALALAQKAREQ GEVPVGAILVLDDEVIGQGWNRPITRHDPTAHAEIMALQQGGQIVQNYRL LNATLYVTFEPCVMCAGAMVHSRIKRLVYGMSNSKRGAAGSLLNVLNYP GMNHQIEITAGVMANECSEMLCQFYRQLRLAPGAEREARRLNQPDRADS GGSSGGSSGSETPGTSESATPESSGGSSGGSPEDNEQKQLFVEQHKHYLDEI IEQISEFSKRVILADANLDKVLSAYNKHRDKPIREQAENIIHLFTLTNLGAPA AFKYFDTTIDRKRYTSTKEVLDATLIHQSITGLYETRIDLSQLGGD |
| | Nucleotide coding sequence of 5V22.2-1249-nCas9 |
| 23 | atgaaacggacagccgacggaagcgagttcgagtcaccaaagaagaagcggaaagtcATGgacaagaagtaca gcatcggcctggccatcggcaccaactctgtgggctgggccgtgatcaccgacgagtacaaggtgcccagcaaga aattcaaggtgctgggcaacaccgaccggcacagcatcaagaagaacctgatcggagccctgctgttcgacagcgg cgaaacagccgaggccacccggctgaagagaaccgccagaagaagatacaccagacggaagaaccggatctgct atctgcaagagatcttcagcaacgagatggccaaggtggacgacagcttcttccacagactggaagagtccttcctgg tggaagaggataagaagcacgagcggcaccccatcttcggcaacatcgtggacgaggtggcctaccacgagaagt accccaccatctaccacctgagaaagaaactggtggacagcaccgacaaggccgacctgcggctgatctatctggc cctggcccacatgatcaagttccggggccacttcctgatcgagggcgacctgaaccccgacaacagcgacgtggac aagctgttcatccagctggtgcagacctacaaccagctgttcgaggaaaaccccatcaacgccagcggcgtggacg ccaaggccatcctgtctgccagactgagcaagagcagacggctggaaaatctgatcgcccagctgcccggcgaga agaagaatggcctgttcggaaacctgattgccctgagcctgggcctgacccccaacttcaagagcaacttcgacctg gccgaggatgccaaactgcagctgagcaaggacacctacgacgacgacctggacaacctgctggcccagatcggc gaccagtacgccgacctgtttctggccgccaagaacctgtccgacgccatcctgctgagcgacatcctgagagtgaa caccgagatcaccaaggcccccctgagcgcctctatgatcaagagatacgacgagcaccaccaggacctgaccctg ctgaaagctctcgtgcggcagcagctgcctgagaagtacaaagagattttcttcgaccagagcaagaacggctacgc cggctacattgacggcggagccagccaggaagagttctacaagttcatcaagcccatcctggaaaagatggacggc accgaggaactgctcgtgaagctgaacagagaggacctgctgcggaagcagcggaccttcgacaacggcagcatc ccccaccagatccacctgggagagctgcacgccattctgcggcggcaggaagatttttacccattcctgaaggacaa ccgggaaaagatcgagaagatcctgaccttccgcatcccctactacgtgggccctctggccaggggaaacagcaga ttcgcctggatgaccagaaagagcgaggaaaccatcaccccctggaacttcgaggaagtggtggacaagggcgctt ccgcccagagcttcatcgagcggatgaccaacttcgataagaacctgcccaacgagaaggtgctgcccaagcacag cctgctgtacgagtacttcaccgtgtataacgagctgaccaaagtgaaatacgtgaccgagggaatgagaaagcccg ccttcctgagcggcgagcagaaaaaggccatcgtggacctgctgttcaagaccaaccggaaagtgaccgtgaagca gctgaaagaggactacttcaagaaaatcgagtgcttcgactccgtggaaatctccggcgtggaagatcggttcaacgc ctccctgggcacataccacgatctgctgaaaattatcaaggacaaggacttcctggacaatgaggaaaacgaggaca ttctggaagatatcgtgctgaccctgacactgtttgaggacagagagatgatcgaggaacggctgaaaacctatgccc acctgttcgacgacaaagtgatgaagcagctgaagcggcggagatacaccggctggggcaggctgagccggaag ctgatcaacggcatccgggacaagcagtccggcaagacaatcctggatttcctgaagtccgacggcttcgccaacag aaacttcatgcagctgatccacgacgacagcctgacctttaaagaggacatccagaaagcccaggtgtccggccag ggcgatagcctgcacgagcacattgccaatctggccggcagccccgccattaagaagggcatcctgcagacagtga aggtggtggacgagctcgtgaaagtgatgggccggcacaagcccgagaacatcgtgatcgaaatggccagagaga accagaccacccagaagggacagaagaacagccgcgagagaatgaagcggatcgaagagggcatcaaagagct gggcagccagatcctgaaagaacaccccgtggaaaacacccagctgcagaacgagaagctgtacctgtactacctg cagaatgggcgggatatgtacgtggaccaggaactggacatcaaccggctgtccgactacgatgtggaccatatcgt gcctcagagctttctgaaggacgactccatcgacaacaaggtgctgaccagaagcgacaagaaccggggcaagag cgacaacgtgccctccgaagaggtcgtgaagaagatgaagaactactggcggcagctgctgaacgccaagctgatt acccagagaaagttcgacaatctgaccaaggccgagagaggcggcctgagcgaactggataaggccggcttcatc aagagacagctggtggaaacccggcagatcacaaagcacgtggcacagatcctggactcccggatgaacactaag tacgacgagaatgacaagctgatccgggaagtgaaagtgatcaccctgaagtccaagctggtgtccgatttccggaa ggatttccagttttacaaagtgcgcgagatcaacaactaccaccacgcccacgacgcctacctgaacgccgtcgtgg gaaccgccctgatcaaaaagtaccctaagctggaaagcgagttcgtgtacggcgactacaaggtgtacgacgtgcg gaagatgatcgccaagagcgagcaggaaatcggcaaggctaccgccaagtacttcttctacagcaacatcatgaact ttttcaagaccgagattaccctggccaacggcgagatccggaagcggcctctgatcgagacaaacggcgaaaccgg ggagatcgtgtgggataagggccgggattttgccaccgtgcggaaagtgctgagcatgccccaagtgaatatcgtga aaaagaccgaggtgcagacaggcggcttcagcaaagagtctatcctgcccaagaggaacagcgataagctgatcg ccagaaagaaggactgggaccctaagaagtacggcggcttcgacagccccaccgtggcctattctgtgctggtggt ggccaaagtggaaaagggcaagtccaagaaactgaagagtgtgaaagagctgctggggatcaccatcatggaaag aagcagcttcgagaagaatcccatcgactttctggaagccaagggctacaaagaagtgaaaaaggacctgatcatca agctgcctaagtactccctgttcgagctggaaaacggccggaagagaatgctggcctctgccggcgaactgcagaa gggaaacgaactggccctgccctccaaatatgtgaacttcctgtacctggccagccactatgagaagctgaagggct ccTCTGGAGGATCTAGCGGCGGATCCTCTGGAAGCGAGACACCAGGCAC |
| | AAGCGAGTCCGCCACACCAGAGAGCTCCGGCGGCTCCTCCGGAGGATC CAGTGAGCTGAATGATGCTTACTGGATGAAACAGGCACTCGCTTTAGCT CAGAAGGCCcggGAACAGGGAGAAGTTCCAGTGGGCGCTATTCTGGTGct gGATGATGAAGTGATAGGACAGGGATGGAATAGACCCATCACCCGGCAC GACCCCACCGCCCACGCCGAGATCATGGCCCTGCAGCAGGGCGGCCAG ATCGTGCAGAACTACCGGCTGCTGAACGCCACCCTGTACGTGACCttcGA GCCCTGCGTGATGTGCGCCGGCGCCATGGTGCACAGCAGGATCAAGAG ACTGGTCTACGGCATGAGCAACtcaAAAagaGGCGCCGCCGGCAGCCTGC TGaacGTGCTGaactacCCCGGCATGAACCACCAGATCGAGATCACCGCCGG CGTGATGGCCAACGAGTGCAGCGAGATGCTGtgcCAGTTCtatCGACAGTT GAGGCTCGCCCCCGGGGCTGAGCGTGAGGCTAGGCGGCTGAACCAACC TGATAGAGCTGACtccggaggatctagcggaggctcctctggctctgagacacctggcacaagcgagag cgcaacacctgaaagcagcgggggcagcagcggggggtcacccgaggataatgagcagaaacagctgtttgtgg aacagcacaagcactacctggacgagatcatcgagcagatcagcgagttctccaagagagtgatcctggccgacgc taatctggacaaagtgctgtccgcctacaacaagcaccgggataagcccatcagagagcaggccgagaatatcatcc acctgtttaccctgaccaatctgggagcccctgccgccttcaagtactttgacaccaccatcgaccggaagaggtaca ccagcaccaaagaggtgctggacgccaccctgatccaccagagcatcaccggcctgtacgagacacggatcgacc tgtctcagctgggaggtgac |
| 24 | Nuclear localization signal (NLS) sequence |
| | KRTADGSEFESPKKKRKV |
| 25 | Nuclear localization signal (NLS) sequence |
| | AVKRPAATKKAGQAKKKKLD |
| 26 | Nuclear localization signal (NLS) sequence |
| | KRPAATKKAGQAKKKK |
| 27 | Nuclear localization signal (NLS) sequence |
| | KKTELQTTNAENKTKKL |
| 28 | Nuclear localization signal (NLS) sequence |
| | KRGINDRNFWRGENGRKTR |
| 29 | Nuclear localization signal (NLS) sequence |
| | RKSGKIAAIVVKRPRK |
| 30 | Nuclear localization signal (NLS) sequence |
| | PKKKRKV |
| 31 | Nuclear localization signal (NLS) sequence |
| | MDSLLMNRRKFLYQFKNVRWAKGRRETYLC |
| 32 | Linker sequence |
| | GGGGS |
| 33 | Linker sequence |
| | SGGS |
| 34 | Linker sequence |
| | SGGSSGSETPGTSESATPESSGGS |
| 35 | Linker sequence |
| | SGGSSGGSSGSETPGTSESATPESSGGSSGGS |
| 36 | Linker sequence |
| | GGSGGSPGSPAGSPTSTEEGTSESATPESGPGTSTEPSEGSAPGSPAGSPTSTE EGTSTEPSEGSAPGTSTEPSEGSAPGTSESATPESGPGSEPATSGGSGGS |
| 37 | Linker sequence |
| | SGSETPGTSESATPES |
| 38 | Linker sequence |
| | SGGSSGGSSGSETPGTSESATPES |
| 39 | Linker sequence |
| | SGGSSGGSSGSETPGTSESATPESSGGSSGGSSGGSSGGS |
| 40 | Linker sequence |
| | SGGSSGGSSGSETPGTSESATPESSGGSSGGSSGGSSGGSSGSETPGTSESAT PESSGGSSGGS |
| 41 | Linker sequence |
| | PGSPAGSPTSTEEGTSESATPESGPGTSTEPSEGSAPGSPAGSPTSTEEGTSTE PSEGSAPGTSTEPSEGSAPGTSESATPESGPGSEPATS |
| 42 | α1-antitrypsin (A1AT) target sequence |
| | ATCGACAAGAAAGGGACTGA |
| 43 | Forward primer for A1AT (A1AT-F) |
| | gacagggagggagaggatgt |
| 44 | Reverse primer for A1AT (A1AT-R) |
| | acagcactgttacctggagc |
| 45 | β-globin target sequence |
| | ACTTCTCCACAGGAGTCAGA |
| 46 | Forward primer for β-globin (β-globin-F) |
| | taagccagtgccagaagagc |
| 47 | Reverse primer for β-globin (β-globin-R) |
| | atagaccaataggcagagag |

Although specific embodiments of the present disclosure have been described above, those skilled in the art should understand that these are merely examples and that various changes or modifications can be made to these embodiments without departing from the principle and essence of the present disclosure. Therefore, the scope of protection of the present disclosure is defined by the appended claims.

## Claims

1. A deaminase comprising the following sequence:
(i) the amino acid sequence as set forth in SEQ ID NO: 10; or
(ii) an amino acid sequence having at least 80%, 82%, 85%, 87%, 90%, 92%, 95%, 96%, 97%, 98% or 99% sequence identity to the amino acid sequence as set forth in SEQ ID NO: 10, which retains the deaminase activity of the deaminase having the amino acid sequence as set forth in SEQ ID NO: 10; and is not SEQ ID NO: 1.

2. The deaminase according to claim 1, wherein the amino acid sequence having at least 80%, 82%, 85%, 87%, 90%, 92%, 95%, 96%, 97%, 98% or 99% sequence identity to the amino acid sequence as set forth in SEQ ID NO: 10 is an amino acid sequence obtained by addition, substitution, deletion or insertion of one or more amino acid residues in the amino acid sequence as set forth in SEQ ID NO: 10;
preferably, the substitution is a substitution occurring at one or more of the following positions in the amino acid sequence as set forth in SEQ ID NO: 10: C46, Y47, G48, H49, C144, Q145, F146, Y147, Q148, Q149, P150, R151, E152, V153, F154, N155, A156, E157, R158, E159, A160, R161, R162, L163, N164, Q165, P166, D167, R168, A169 and D170;
more preferably, the substitution is a substitution occurring in a combination of the following positions in the amino acid sequence as set forth in SEQ ID NO: 10:
(1) 7 or 10 of C46, Y47, G48, H49, Q148, P150, E152, V153, F154 and N155;
(2) 6, 7 or 8 of Q148, Q149, P150, R151, E152, V153, F154 and N155;
(3) 7 or 8 of A156, E157, R158, E159, A160, R161, R162 and L163;
(4) 6 or 7 of N164, Q165, P166, D167, R168, A169 and D170;
(5) 6 or 7 of C144, Q145, F146, Y147, Q148, Q149 and P150; or
(6) 2 or 9 of C144, Q145, Q148, Q149, P150, E152, V153, F154 and N155.

3. The deaminase according to claim 2, wherein the substitution is a substitution occurring in any one of the combination of the following positions in the amino acid sequence as set forth in SEQ ID NO: 10:
(1) C46, Y47, G48, H49, Q148, P150, E152, V153, F154 and N155;
(2) G48, Q148, P150, E152, V153, F154 and N155;
(3) Q148, Q149, P150, E152, V153, F154 and N155;
(4) Q148, P150, E152, V153, F154 and N155;
(5) Q149, P150, R151, E152, V153, F154 and N155;
(6) Q148, Q149, P150, R151, E152, V153, F154 and N155;
(7) Q148, Q149, P150, E152, F154 and N155;
(8) A156, E157, R158, E159, A160, R161, R162 and L163;
(9) A156, E157, R158, E159, A160, R162 and L163;
(10) N164, Q165, P166, D167, R168, A169 and D170;
(11) N164, Q165, D167, R168, A169 and D170;
(12) C144, Q145, F146, Y147, Q148, Q149 and P150;
(13) C144, Q145, F146, Y147, Q148 and P150;
(14) C144, Q145, Q148, Q149, P150, E152, V153, F154 and N155;
(15) C144 and Q145;
preferably, the substitution occurring at the positions is selected from: C46P, Y47I, G48A/T, H49R, C144T/L/W, Q145L/K, F146A/R, Y147S/F, Q148R/G/T/S/C, Q149N/P/R/V/G/F/C/K, P150A/L/R/I/G/S/T, R151K/P, E152P/L/Q/H/S, V153T/A/F/Y/K/P, F154S/P/V/N/L/D/H, N155P/G/T/S/Y/R/A, A156L/T, E157F, R158N/L, E159L/H, A160K/T, R161K, R162L/K, L163D/I, N164G/R, Q165T/L, P166Q, D167L, R168L/P, A169N/T and D170R/H; preferably selected from: C46P, Y47I, G48A/T, H49R, C144T/L/W, Q145L/K, F146A/R, Y147S/F, Q148R/T/C, Q149N/P/R/G/C/K, P150A/L/R/G/S/T, R151P, E152P/L/Q/H, V153T/A/F/K, F154S/P/V/L/D/H, N155P/G/T/S/A, A156L/T, E157F, R158N/L, E159L/H, A160K/T, R161K, R162L/K, L163D/I, N164G/R, Q165T/L, P166Q, D167L, R168L/P, A169N/T and D170R/H;
more preferably, the substitution is a substitution occurring in a combination of the following positions in the amino acid sequence as set forth in SEQ ID NO: 10:
(1) Q148R, Q149N, P150A, E152P, V153T, F154S, N155P;
(2) Q148R, P150L, E152L, V153A, F154P, N155G;
(3) Q148R, Q149R, P150R, E152P, V153F, F154V, N155T;
(4) Q148G, Q149G, P150I, E152L, V153Y, F154N, N155S;
(5) Q149P, P150G, R151K, E152Q, V153K, F154L, N155P;
(6) Q148R, Q149V, P150S, R151P, E152L, V153F, F154P, N155Y;
(7) Q148T, Q149G, P150R, E152H, V153A, F154D, N155S;
(8) Q148S, Q149F, P150L, E152S, V153P, F154L, N155R;
(9) Q148G, Q149C, P150S, E152P, F154H, N155A;
(10) A156L, E157F, R158N, E159L, A160K, R161K, R162L, L163D;
(11) A156T, E157F, R158L, E159H, A160T, R162K, L163I;
(12) N164G, Q165T, P166Q, D167L, R168L, A169N, D170R;
(13) N164R, Q165L, D167L, R168P, A169T, D170H;
(14) C144T, Q145L, F146A, Y147S, Q148R, Q149K, P150S;
(15) C144L, Q145L, F146R, Y147F, Q148C, P150T;
(16) C144W, Q145K, Q148R, Q149R, P150R, E152P, V153F, F154V, N155T;
(17) C144W, Q145K;
(18) G48A, Q148R, P150L, E152L, V153A, F154P, N155G;
(19) C46P, Y47I, G48T, H49R, Q148R, P150L, E152L, V153A, F154P, N155G.

4. A base editor fusion protein comprising the deaminase according to any one of claims 1 to 3, and a nucleic acid-programmable nucleotide-binding domain;
preferably, the nucleic acid-programmable nucleotide-binding domain is a Cas protein or an AGO protein; the Cas protein is e.g., selected from Cas9, CasX, CasY, Cpf1, C2c1, C2c2 and C2c3; and/or the AGO protein is e.g., selected from pAgo, eAgo, Ago1, Ago2, Ago3 and Ago4;
and/or, the deaminase is linked to one terminus of the nucleic acid-programmable nucleotide-binding domain or is chimeric within the nucleic acid-programmable nucleotide-binding domain;
more preferably, the linkage is a direct linkage or a linkage via a linker; and/or, the chimeric site is located in the carboxy-terminal domain of the nucleic acid-programmable nucleotide-binding domain; and/or, said nucleic acid-programmable nucleotide-binding domain retains part or none of the cleavage activity on a nucleotide strand; the linker preferably comprises an amino acid sequence as set forth in one or more of SEQ ID NOs: 32-41.

5. The base editor fusion protein according to claim 4, wherein the base editor fusion protein further comprises a nuclear localization signal (NLS) sequence; the NLS sequence is linked to the N-terminus and/or C-terminus of the base editor fusion protein, and/or, to the N-terminus and/or C-terminus of the deaminase;
preferably, the NLS sequence is linked to both the N-terminus and the C-terminus of the base editor fusion protein; for example, the structure of the base editor fusion protein from N-terminus to C-terminus is: NLS sequence-deaminase-nucleic acid-programmable nucleotide-binding domain-NLS sequence; or NLS sequence-deaminase-nucleic acid-programmable nucleotide-binding domain-NLS sequence.

6. The base editor fusion protein according to claim 4 or 5, wherein when the nucleic acid-programmable nucleotide-binding domain is a Cas protein, e.g., a Cas9 protein, the chimeric site is located between positions 1249 and 1250 of Cas9;
preferably, the base editor fusion protein comprises an amino acid sequence as set forth in any one of SEQ ID NOs: 10, 18, 20 and 22.

7. A base editing system comprising:
(i) the deaminase according to any one of claims 1 to 3 and a nucleic acid-programmable nucleotide-binding domain;
or (ii) the base editor fusion protein according to any one of claims 4 to 6,
and a guide polynucleotide;
wherein the nucleic acid-programmable nucleotide-binding domain or the base editor fusion protein forms a ribonucleoprotein complex with the guide polynucleotide, and binds to a target nucleic acid under the guidance of the guide polynucleotide.

8. A polynucleotide encoding the deaminase according to any one of claims 1 to 3, the base editor fusion protein according to any one of claims 4 to 6, or the base editing system according to claim 7;
preferably, the polynucleotide encoding said base editor fusion protein comprises a nucleotide sequence as set forth in any one of SEQ ID NOs: 11, 19, 21 and 23.

9. A vector comprising the polynucleotide according to claim 8;
preferably, the polynucleotide is located on one or more vectors; and/or, the vector further comprises a promoter, and the polynucleotide is operably linked to the promoter;
more preferably, the promoter is one or more selected from a constitutive promoter, an inducible promoter, a ubiquitin promoter, a cell type-specific promoter and a tissue-specific promoter.

10. An isolated cell comprising the deaminase according to any one of claims 1 to 3, the base editor fusion protein according to any one of claims 4 to 6, the polynucleotide according to claim 8 and/or the vector according to claim 9;
preferably, the cell is a prokaryotic cell or a eukaryotic cell; e.g., selected from an animal cell, a plant cell and a fungal cell;
more preferably, the cell is a vertebrate cell or an invertebrate cell; the vertebrate cell is preferably a mammalian cell;
even more preferably, the mammalian cell is selected from a rodent cell, a primate cell and a non-primate cell; the primate cell is e.g., a human cell.

11. A pharmaceutical composition comprising the deaminase according to any one of claims 1 to 3, the base editor fusion protein according to any one of claims 4 to 6, the base editing system according to claim 7, the polynucleotide according to claim 8, the vector according to claim 9 and/or the cell according to claim 10, and optionally a pharmaceutically acceptable carrier and/or excipient.

12. A kit comprising the deaminase according to any one of claims 1 to 3, the base editor fusion protein according to any one of claims 4 to 6, the base editing system according to claim 7, the polynucleotide according to claim 8, the vector according to claim 9, the cell according to claim 10 and/or the pharmaceutical composition according to claim 11.

13. A delivery system comprising the deaminase according to any one of claims 1 to 3, the base editor fusion protein according to any one of claims 4 to 6, the base editing system according to claim 7, the polynucleotide according to claim 8, the vector according to claim 9, the cell according to claim 10, the pharmaceutical composition according to claim 11 and/or the kit according to claim 12, and a delivery vehicle;
preferably, said delivery vehicle is selected from liposomes, nanoparticles, viral vectors, exosomes, microvesicles and cell-penetrating peptides.

14. A method for base editing, comprising the step of contacting a target nucleic acid with the deaminase according to any one of claims 1 to 3, the base editor fusion protein according to any one of claims 4 to 6, or the base editing system according to claim 7 to effect a deamination reaction;
preferably, the base editing method is performed *in vivo* or in *vitro*; and/or, the method for base editing is for non-diagnostic or non-therapeutic purposes.

15. Use of the deaminase according to any one of claims 1 to 3, the base editor fusion protein according to any one of claims 4 to 6, the base editing system according to claim 7, the polynucleotide according to claim 8, the vector according to claim 9, the cell according to claim 10, the pharmaceutical composition according to claim 11, the kit according to claim 12, or the delivery system according to claim 13 in the manufacture of a medicament for treating a disease associated with or caused by a point mutation;
preferably, the disease is one or more selected from hypercholesterolemia, transthyretin amyloidosis, alpha-1 antitrypsin deficiency, and beta-hemoglobinopathies.
